# EUROPEAN PATENT SPECIFICATION

(11) **EP 1 928 571 B1**
(45) Date of publication and mention of the grant of the patent: **13.03.2013**
(21) Application number: 06802140.1
(22) Date of filing: 22.08.2006
(51) Int. Cl.: F15C 3/00, B01L 3/00

(54) **DEVICE AND METHOD FOR MICROFLUIDIC CONTROL OF A FIRST FLUID IN CONTACT WITH A SECOND FLUID, WHEREIN THE FIRST AND SECOND FLUIDS ARE IMMISCIBLE**
VORRICHTUNG UND VERFAHREN ZUR MIKROFLUIDISCHEN STEUERUNG EINES ERSTEN FLUIDS IN KONTAKT MIT EINEM ZWEITEN FLUID, WOBEI DAS ERSTE FLUID UND DAS ZWEITE FLUID NICHT MISCHBAR SIND
DISPOSITIF ET PROCEDE DE COMMANDE D'UN PREMIER FLUIDE EN CONTACT AVEC UN SECOND FLUIDE, CE PREMIER ET CE SECOND FLUIDE ETANT IMMISCIBLES

(30) Priority: 22.08.2005 US 710167 P; 28.10.2005 US 731133 P; 30.06.2006 US 818197 P
(43) Date of publication of application: 11.06.2008
(73) Proprietor: Life Technologies Corporation, Carlsbad, CA 92008 (US)
(72) Inventor: COX, David M., Foster City, California 94404 (US); WOO, Sam Lee, Redwood City, California 94061 (US); BOEGE, Steven J., San Mateo, California 94403-5030 (US); OLDHAM, Mark F., Los Gatos, California 95033 (US); REEL, Richard T., Hayward, California 94541-2451 (US); NORDMAN, Eric S., Palo Alto, California 94301 (US); LEE, Linda G., Palo Alto, California 94306 (US); WIYATNO, Willy, Union City, California 94587 (US); SAGATELYAN, Dmitry M., Alameda, California 94502 (US)
(74) Representative: Wöhler, Christian
(86) International application number: PCT/US2006/032861
(87) International publication number: WO 2007/024914

(56) References cited:
- EP-A2- 0 562 260
- WO-A1-03/076052
- DE-A1- 10 322 893
- US-A- 5 083 872
- US-A- 5 247 957
- US-A- 5 927 326
- US-A1- 2001 016 177
- US-A1- 2003 044 832
- US-A1- 2005 087 122
- LINK D R ET AL: "Geometrically Mediated Breakup of Drops in Microfluidic Devices", PHYSICAL REVIEW LETTERS, AMERICAN PHYSICAL SOCIETY, NEW YORK, US, vol. 92, no. 5, 6 February 2004 (2004-02-06), pages 54503-1, XP007917115, ISSN: 0031-9007, DOI: DOI:10.1103/PHYSREVLETT.92.054503

## Description

The section headings used herein are solely for organization purposes and are not to be construed as limiting the subject matter described in any way.

Large scale sequencing projects can involve cloning DNA fragments in bacteria, picking and amplifying those fragments, and performing individual sequencing reactions on each clone. Standard sequencing reactions can often be performed in 5 µ1 to 20 µ1 reaction volumes, even though only a small fraction of the sequencing product can be analyzed. Such cloning and sequencing protocols can be time consuming and can use relatively large sample and reagent volumes. The relatively large volumes can be wasteful in terms of expensive consumable reagents.
An article of D.R. Link et al in Physical Review Letters, vol. 92, no. 5, pages 54053-1 to 54053-4 (6 February 2004) discloses passively breaking larger drops into precisely controlled smaller drops using pressure-driven flow through a T junction. The relative sizes of different daughter drops are reported to correspond to the relative arm lengths of different conduits of the T junction.

### SUMMARY

Various embodiments of the present teachings relate to systems, apparatus, and/or methods for sample preparation that can be used for biochemical or molecular biology procedures involving different volumes, for example, small volumes such as micro-liter sized volumes or smaller. The invention concerns a method according to to claim 1 and a device according to claim 5, with preferred embodiments described by the dependent claims.

According to the present teachings, the system can comprise an apparatus for generating discrete volumes of at least a first fluid in contact with a second fluid, wherein the first and second fluids are immiscible with each other, for example, discrete volumes of an aqueous liquid (herein "aqueous immiscible-fluid-discrete-volumes"), spaced-apart from one another by a spacing fluid that is immiscible with the immiscible-fluid-discrete-volumes. An immiscible-fluid-discrete-volume can be a partitioned segment in which molecular biology procedures can be performed. As used herein, an immiscible-fluid-discrete-volume can be one of many structures, three of which are: a fluid segment, a slug, and an emulsified droplet. In some embodiments, immiscible-fluid-discrete-volumes are formed and/or processed in a conduit.

This paragraph defines a conduit as it is used herein. A conduit can be any device in which an immiscible-fluid-discrete-volume can be generated, conveyed, and/or flowed. For example, a conduit as defined herein can comprise any of a duct, a tube, a pipe, a channel, a capillary, a hole or another passageway in a solid structure, or a combination of two or more of these, as long as the spaces defined by the respective solid structures are in fluid communication with one another. A conduit can comprise two or more tubes or other passageways connected together, or an entire system of different passageways connected together. An exemplary conduit can comprise an immiscible-fluid-discrete-volume-forming tube, thermal spirals, valve passageways, a processing conduit, junctions, and the like components all connected together to form one or more fluid communications therethrough, which system is also referred to herein as a main processing conduit. Examples of solid structures with holes or passageways therein that can function as conduits are manifolds, T-junctions, Y-junctions, rotary valves, and other valves. Thus, when connected to conduits, such structures can be considered part of a conduit as defined herein.

This paragraph defines a fluid segment, as it is used herein. A fluid segment is a discrete volume that has significant contact with one or more conduit wall(s), such that a cross-sectional area of the fluid segment is the same size and shape as the cross-sectional area of the conduit it contacts. At least a portion of a fluid segment fully fills the cross-sectional area of the conduit, such that the immiscible fluid adjacent it in the conduit can not flow past the fluid segment. The entire longitudinal length of the fluid segment may not contact the conduit walls.

This paragraph defines a slug as used herein. A slug is a discrete volume that has at least a portion of which has approximately the same cross-sectional shape as the conduit in which it exists, but a smaller size. The smaller size is due to the insignificant contact, if any, of the slug with the conduit wall(s). A slug can have a cross-sectional dimension between approximately 0.5 and approximately 1.0 times the maximum dimension of a cross sectional area of the conduit. If the conduit has a circular cross section, the cross-sectional area of a slug can be concentric with the conduit's cross-sectional area, but it does not have to be, such as, for example, when the conduit is horizontal and, due to different specific gravities, one fluid rises toward the top of the cross-sectional area of the conduit under the influence of gravity. A slug can be free of contact with the conduit walls. When not moving relative to the conduit, a slug can have "feet" that appear as nibs or bumps along an otherwise smoothly appearing round surface. It is theorized that the feet at the bottom of the slug may have contact with the conduit wall. In contrast to a fluid segment, the contact a slug can have with the conduit wall(s) still permits the immiscible fluid adjacent it in the conduit to flow past the slug.

The "slugs" formed by the teachings herein, separated by spacing fluid, can merge together to form larger slugs of liquid, if contacted together. The ability of the slugs, for example, aqueous slugs, described and taught herein, to merge together with each other, facilitates the downstream addition of aqueous reagents to the slugs.

This paragraph defines an emulsified droplet, as used herein. An emulsified droplet is a discrete volume that has no contact with the walls of the conduit. The size of an emulsified droplet is not necessarily constrained by the conduit, and examples of emulsified droplets described in the prior art range in size from about 1 femtoliter to about 1 nanoliter. The shape of an emulsified droplet is not constrained by the conduit, and due to the difference in surface-energies between it and the continuous phase liquid in which it is dispersed, it is generally spherical. It can have a maximum dimension that is not equal to, nor approximately equal to, but much less than the maximum dimension of the cross-sectional area of the conduit, for example, 20%, 10%, 5% or less. An emulsified droplet will not merge upon contact with another emulsified droplet to form a single, larger discrete volume, without external control. Put another way, an emulsified droplet is a stable discontinuous phase in a continuous phase.

A conduit can contain more than one emulsified droplet, but not more than one slug or fluid segment, at any cross-sectional location. Thus, a first emulsified droplet does not necessarily impede the movement of a second emulsified droplet past it, where as a fluid segment and a slug necessarily do not permit the passage of another fluid segment or slug past them, respectively. If two fluid segments are separated by a fluid with which the first and second of the two fluids is each immiscible, then the immiscible fluid also forms a discrete volume. It is likely that it has significant contact with the conduit walls and thus is another fluid segment.

Whether two immiscible fluids, when present in a conduit, form fluid segments of the first and second of the two immiscible fluids, slugs of the first immiscible fluid, or emulsified droplets of the first immiscible fluid depends on at least the method of introduction of each fluid into the conduit, the relative surface energies of the first immiscible fluid, the second immiscible fluid, and the conduit material, the contact angle each forms with the other two materials, respectively, and the volume of the discrete volume of the first immiscible fluid. Thus, it is recognized that these definitions are merely reference points on a continuum, the continuum of the shape and size of discrete volumes of a first immiscible fluid in a conduit, and discrete volumes will exist that, when described, fall between these definitions.

The molecular biology procedures can, for example, utilize proteins or nucleic acids. Procedures with nucleic acids can comprise, for example, a PCR amplification and/or nucleic acid analysis of an amplification product. The PCR amplification and/or nucleic acid analysis of an amplification product can comprise an integrated DNA amplification/DNA sequencing method.

Using the apparatus, methods, and/or systems provided in this application, a polymerase chain reaction (PCR) amplification of single DNA molecules can be performed, for example, to obtain amplicons. The amplified DNA or amplicons can then be used in a sequencing reaction and then be sequenced in small volumes. Other manipulations of nucleic acids or proteins can also be accomplished, for example, DNA hybridization reactions or antibody-antigen binding assays.

An exemplary type of sample preparation can be used for genotyping, gene-expression, methylation analysis, and/or directed medical sequencing (VariantSEQr™, for example, an Applied Biosystems product comprising primers for resequencing genes and detecting variations) that requires multiple liquids to be brought together in an aqueous discrete volume. For example, in a gene-expression application, each aqueous discrete volume can contain individual primer sets. The sample to be analyzed, for example, complementary DNA (cDNA), can be added to each aqueous discrete volume. In the VariantSEQr™ application, for example, an aqueous discrete volume can comprise a primer set and genomic DNA can be added to that discrete volume. According to various embodiments, a system and method are provided that are able to generate discrete volumes with unique content. According to various embodiments of the present teachings, sipping, other aspirating, or other techniques to generate immiscible-liquid, discrete volumes can be used. According to various embodiments, an immiscible-liquid, discrete volume of at least an aqueous sample fluid can be generated in a tube by alternately drawing into the tube the aqueous sample fluid and spacing fluid, with which the aqueous sample fluid is immiscible, from a single container or well containing both fluids or from different containers or wells each containing one of the two fluids.

Using the apparatus, methods, and/or systems provided in this application, one can control the positioning of immiscible-fluid, discrete volumes during processing. These processes can include, for example, polymerase chain reaction (PCR) amplification of single DNA molecules to obtain, for example, amplicons. The amplified DNA or amplicons can then be used in a sequencing reaction and be sequenced using small volumes. Other manipulations of nucleic acids or proteins can also be accomplished, for example, DNA hybridization reactions or antibody-antigen binding assays.

In some embodiments, flow rates for moving aqueous discrete volumes can comprise rates of from about 1 picoliter/sec. to about 200 microliters/sec., and can be selected based on the inner diameter of the conduits through which the liquids are to be pumped. Within that broad range, in some embodiments, the aqueous discrete volumes comprising 50% reagents, for example, PCR reagents, and 50% other reagents, for example, sample fluid, in contact with oil can flow at 0.5 microliter/sec, or in a range from about 0.1 microliter/sec. to 2 microliters/sec. In some embodiments, aqueous volumes comprising reagents, for example, exo SAP or sequencing mix, can flow at a rate of about 1/3 microliters/sec., or in a range from about 0.1 microliters/sec. to 1 microliters/sec. In some embodiments, when aspirating aqueous fluid into a conduit as discrete volumes, flow rates of up to about 0.1 microliters/sec. max can be used. In some embodiments, when outputting an aqueous discrete volume, a flow rate of about 1/3 to about 1/2 µl/sec can be used, or in a range from about 0.1 microliters/sec. to about 2 microliters/sec. can be used.

Tubing that can be used with the 1 picoliter/sec. to 200 microliter/sec. flow rate can comprise an inner diameter of from about 250 microns to about 1000 microns. In other embodiments, the inner diameter of the inner tube can be from about 10 microns to about 2000 microns, while the inner diameter of the outer tube can be from about 20 microns to about 5000 microns, for example, from about 35 microns to about 500 microns. Other diameters, however, can be used based on the characteristics of the slug processing system desired. In some embodiments, a tube having a 10 micron inner diameter is used with a flow rate of from about 8 to about 10 picoliters/second. In some embodiments, a tube having a 5000 micron inner diameter is used with a flow rate of from about 25 to about 200 microliters/second. In some embodiments, a tube having a 500 micron inner diameter is used with a flow rate of from about 0.25 to about 2.0 microliters/second.

The aqueous sample liquid, which in some embodiments can form discrete volumes in contact with a fluid with which it is immiscible, can comprise a plurality of target nucleic acid sequences, wherein at least one of the discrete volumes comprises at least one target nucleic acid sequence. In some embodiments, after formation, at least 37% of the plurality of the discrete volumes in the inner conduit can each comprise a single target nucleic acid sequence. In various other embodiments, less than about 37% of the plurality of discrete volumes in the conduit can each comprise a single target nucleic acid sequence. In other embodiments, at least 1% or more, 5% or more, 10% or more, or 20% or more can have a single target nucleic acid sequence, for example, upon formation of the discrete volumes.

According to various embodiments, each of the plurality of discrete volumes in the inner conduit can comprise one or more respective oligonucleotide primers. Oligonucleotide primers can be chosen as determined by one of skill in the art to accomplish the desired objective. For example, universal primers can be used.

In some embodiments, further downstream processing of the prepared immiscible-fluid-discrete-volumes can be integrated with the system, of which embodiments are described herein. Such downstream processing can include amplifying the at least one target nucleic acid sequence in the first discrete volume in the conduit to form an amplicon, and thereafter subjecting the amplicon to a nucleic acid sequencing reaction. For such purposes, and in some embodiments, the discrete volumes or immiscible-fluid-discrete-volumes can comprise reaction components, for example, oligonucleotide primers. Various embodiments of downstream processing can include universal PCR, or can comprise up-front multiplexed PCR followed by decoding, for example, see WO 2004/051218 to Andersen et al., U.S. Patent No. 6,605,451 to Marmaro et al., U.S. Patent Application No. 11/090,830 to Andersen et al., and U.S. Patent Application No. 11/090,468 to Lao et al. Details of real time PCR can be found in Higuchi et al., U.S. Patent No. 6,814,934 B1.

### DRAWINGS

The skilled artisan will understand that the drawings described below are for illustrative purposes only. In the drawings:

FIGS. 1A and 1B illustrate a system using microfluidic immiscible-fluid, discrete volumes in which nucleic acid assays can be performed;

FIG. 2 illustrates a system using microfluidic immiscible-fluid, discrete volumes in which nucleic acid assays can be performed ;

FIG. 3 illustrates a cross-sectional view in the XY plane of an embodiment of a rotary valve;

FIG. 4 illustrates a cross-sectional view in the XY plane of another embodiment of a rotary valve; the rotor of the rotary valve having a two ducts therethrough one of which is aligned with a duct through the stator; and

FIG. 5 illustrates a cross-sectional view in the XY plane of the rotary value of FIG. 4, where the relative positions of the rotor and stator have changed such that the second of the two parallel ducts is in fluid communication with the first and third ducts through the stator;

FIG. 6 illustrates a cross-sectional view in the XY plane of an embodiment of a linear valve in the open position;

FIG. 7 illustrates a cross-sectional view in the XY plane of the linear valve of FIG. 6 in a closed position;

FIG. 8 illustrates a side view of an embodiment of a pinch valve in an open position;

FIG. 9 illustrates a front, cross-sectional view of the pinch valve of FIG. 8;

FIG. 10 illustrates a front, cross-sectional view of the pinch valve of FIG. 8, but in a closed position;

FIG. 11A illustrates an embodiment of a system that can be used to (1) combine two miscible fluid streams in preset volumetric ratios, (2) split an immiscible-fluid, discrete volume into two smaller, distinct, discrete volumes, (3) generate immiscible-fluid, discrete volumes of a first fluid in a consistent ratio with a second fluid with which it is immiscible and (4) combine a first immiscible-fluid, discrete volume with a second immiscible-fluid, discrete volume;

FIG. 11B illustrates the system of FIG. 11A, except the three-way valves are illustrated with the common port of each connected to the second of the two input/output ports and the displacement pumps have been moved in the opposite direction;

FIG. 12 illustrates an embodiment of a slug splitter with a slug in position in the supply tube to split;

FIG. 13 illustrates the slug splitter with a first of the two portions of the split slug in fluid communication with the first-split-portion conduit;

FIG. 14 illustrates the slug splitter after the first of two portions of the slug has flowed into the first-split-portion tube and spacing fluid has flowed into the slider through-hole;

FIG. 15 illustrates the slug splitter with the spacing fluid in the slider in fluid communication with the second-split-portion conduit;

FIG. 16 illustrates advancing the second of two portions of the split slug through the slider and into the second-split-portion conduit;

FIG. 17 illustrates an embodiment of a bubble remover;

FIG. 18 illustrates another embodiment of a bubble remover;

FIG. 19 illustrates an embodiment of an apparatus for pressurizing an immiscible-fluid-discrete-volume processing system;

FIG. 20 illustrates another embodiment of an apparatus for pressuring an immiscible-fluid-discrete-volume processing system;

FIG. 21 illustrates a system of fluidic control using optical detectors to provide feedback;

FIG. 22 illustrates an embodiment of an inspection system;

FIG. 23 illustrates using a "T" junction to add liquid to a conduit containing aqueous discrete volumes, with a constant flow rate of the "addition" liquid matched to add a pre-determined volume of liquid either before or after an aqueous discrete volume or to pre-existing aqueous discrete volumes moving past the junction at a known speed;

FIG. 24 illustrates using a "T" junction to add discrete volumes of aqueous fluid in one of the vertical conduits to one or more discrete volumes of aqueous fluid in a main conduit, the flow rates and spacing of the immiscible-fluid, discrete volumes in each conduit being controlled to ensure coalescence;

FIG. 25 illustrates using a dedicated displacement pump for each junction of an addition tube and an immiscible-fluid-discrete-volume supply tube;

FIG. 26 illustrates a system using a single pressure pump to positively pressurize liquid supplies in addition tubes to multiple T-junctions, and a valve for each addition tube to meter a known volume of liquid to the immiscible-fluid-discrete-volume supply tube;

FIG. 27 uses a single pressure pump to positively pressurize all liquid supply vessels and, as a result, the addition tube to each individual T-junction, and a valve to meter the amount of liquid added to the immiscible-fluid-discrete-volume supply tube;

FIG. 28 illustrates using a T-junction and valving to merge two streams of immiscible-fluid, discrete volumes into a stream of larger-volume, immiscible-fluid discrete volume;

FIG. 29A-C illustrates using adding microdroplets (e.g., stable emulsified nanodroplets) of aqueous liquid using a T to merge with slugs in main tube after being held by a field and then expelled from vertical tube into the main conduit;

FIG. 30 illustrates a system for moving sets of immiscible-fluid, discrete volumes to a storage tube under vacuum before introducing them under positive pressure to the main conduit system of a immiscible-fluid-discrete-volume processing system;

FIG. 31 illustrates the positions of sets of immiscible-fluid, discrete volumes in an apparatus during steps of a method for manipulating them into a processing system; and

FIG. 32 illustrates the positions of sets of immiscible-fluid, discrete volumes in an apparatus during steps of another method for manipulating them into a processing system.

### DESCRIPTION OF VARIOUS EMBODIMENTS

It is to be understood that the following descriptions are exemplary and explanatory only. The accompanying drawings are incorporated in and constitute a part of this application and illustrate several exemplary embodiments with the description. Reference will now be made to various embodiments, examples of which are illustrated in the accompanying drawings. Wherever possible, the same reference numbers are used in the drawings and the description to refer to the same or like parts.

Throughout the application, descriptions of various embodiments use "comprising" language, however, it will be understood by one of skill in the art, that in some specific instances, an embodiment can alternatively be described using the language "consisting essentially of" or "consisting of."

For purposes of better understanding the present teachings and in no way limiting the scope of the teachings, it will be clear to one of skill in the art that the use of the singular includes the plural unless specifically stated otherwise. Therefore, the terms "a," "an" and "at least one" are used interchangeably in this application.

Unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. In some instances, "about" can be understood to mean a given value + 5%. Therefore, for example, about 100 n1, could mean 95-105 n1. At the very least, each numerical parameter should at least be construed in light of the number of reported significant digits and by applying ordinary rounding techniques.

Reference to "nucleotide" should be understood to mean a phosphate ester of a nucleotide, as a monomer unit or within a nucleic acid. Nucleotides are sometimes denoted as "NTP", or "dNTP" and "ddNTP" to particularly point out the structural features of the ribose sugar. "Nucleotide 5'-triphosphate" can refer to a nucleotide with a triphosphate ester group at the 5' position. The triphosphate ester group may include sulfur substitutions for the various oxygens, for example, α-thio-nucleotide 5'-triphosphates. Nucleotides can comprise a moiety of substitutes, for example, see, U.S. Patent No. 6,525,183 B2 to Vinayak et al.

The terms "polynucleotide" or "oligonucleotide" or "nucleic acid" can be used interchangeably and include single-stranded or double-stranded polymers of nucleotide monomers, including 2'-deoxyribonucleotides (DNA) and ribonucleotides (RNA) linked by internucleotide phosphodiester bond linkages, or internucleotide analogs, and associated counter ions, for example, H+, NH4+, trialkylammonium, Mg2+, Na+ and the like. A polynucleotide may be composed entirely of deoxyribonucleotides, entirely of ribonucleotides, or chimeric mixtures thereof. Polynucleotides may be comprised of nucleobase and sugar analogs. Polynucleotides typically range in size from a few monomeric units, for example, 5-40 when they are frequently referred to in the art as oligonucleotides, to several thousands of monomeric nucleotide units. Unless denoted otherwise, whenever a polynucleotide sequence is represented, it will be understood that the nucleotides are in 5' to 3' order from left to right and that "A" denotes deoxyadenosine, "C" denotes deoxycytidine, "G" denotes deoxyguanosine, and "T" denotes thymidine, unless otherwise noted. A labeled polynucleotide can comprise modification at the 5'terminus, 3'terminus, a nucleobase, an internucleotide linkage, a sugar, amino, sulfide, hydroxyl, or carboxyl. See, for example, U.S. Patent No. 6,316,610 B2 to Lee et al. Similarly, other modifications can be made at the indicated sites as deemed appropriate.

The term "reagent," should be understood to mean any reaction component that in any way affects how a desired reaction can proceed or be analyzed. The reagent can comprise a reactive or non-reactive component. It is not necessary for the reagent to participate in the reaction. The reagent can be a recoverable component comprising for example, a solvent and/or a catalyst. The reagent can comprise a promoter, accelerant, or retardant that is not necessary for a reaction but affects the reaction, for example, affects the rate of the reaction. A reagent can comprise, for example, one member of a binding pair, a buffer, or a DNA that hybridizes to another DNA. The term "reagent" is used synonymous with the term "reaction component."

Methods, apparatuses and systems described herein can use fluids immiscible in each other. Fluids can be said to be immiscible in each other when they can be maintained as separate fluid phases under conditions being used. Immiscible fluids can also be said to be incapable of mixing with each other or attaining a solution with each other. An aqueous liquid and a non-aqueous liquid such as oil can be said to be immiscible with each other. Throughout the specification, reference is made to aqueous slugs. This is merely exemplary and does not necessarily preclude the use or manufacture of non-aqueous liquid slugs in combination with an immiscible liquid.

While oil and aqueous liquids are immiscible in each other, such a combination does not necessarily form aqueous immiscible-fluid-discrete-volumes in the oil when the two liquids are mixed or placed together. For example, oil may form the disperse phase in a continuous aqueous liquid in a larger volume, as it does in certain salad dressings. For another example, oil and aqueous liquids may merely form aqueous droplets or microdroplets in a larger volume of oil, but not necessarily aqueous immiscible-fluid-discrete-volumes.

Aqueous solutions and oil from separate sources can be combined to form a continuous flowing liquid stream comprising aqueous immiscible-fluid-discrete-volumes separated from one another by the oil. Because the aqueous immiscible-fluid-discrete-volumes entirely or almost entirely fill the cross-sectional area of the conduit or tube in which they are formed, the resulting stream of aqueous immiscible-fluid-discrete-volumes in oil can exhibit a banded appearance. According to various embodiments, such a pattern can be exhibited by combining any two immiscible fluids with one another. The pattern can be formed throughout the length of the conduit. In various embodiments, a first aqueous immiscible-fluid-discrete-volume can contain different reagents than a second aqueous immiscible-fluid-discrete-volume. In other words, not all aqueous immiscible-fluid-discrete-volumes throughout the conduit need to contain the same reagents.

An aqueous immiscible-fluid-discrete-volume can be spaced apart from an adjacent aqueous immiscible-fluid-discrete-volume by the oil. In various embodiments, liquids other than oil can act as a spacing fluid, provided that the spacing fluid and aqueous fluid are immiscible with respect to each other and provided that they can form individual aqueous immiscible-fluid-discrete-volumes spaced apart from one another by the spacing fluid. In various embodiments, gas can be used as a spacing fluid.

According to various embodiments, methods are provided that refer to processes or actions involved in sample preparation and analysis. It will be understood that in various embodiments a method can be performed in the order of processes as presented, however, in related embodiments, the order can be altered as deemed appropriate by one of skill in the art in order to accomplish a desired objective.

According to various embodiments, an apparatus is provided that can be used as a front-end sample preparation device for high-throughput sequencing, or other applications requiring preparation and/or processing of a plurality of small samples. The sample liquid that can become an immiscible-fluid-discrete-volume can comprise, for example, nucleic acids, proteins, polypeptides, carbohydrates, or the like. The apparatus can be part of an integrated system and/or be adapted to function with other pieces of equipment adapted for further sample processing of samples, for example, an ABI 310, ABI 3130, ABI 3130x1, ABI 3700, ABI 3730, or ABI 3730x1 capillary electrophoresis analyzer (available from Applied Biosystems, Foster City, California) that can be used for sequencing. In some embodiments, the apparatus can be part of an integrated system and/or be adapted to function with other pieces of equipment adapted for further sample processing of samples, for example, a PCR detector. Exemplary detectors that can be used include real-time sequence detection systems and real-time PCR detectors, for example, the ABI 7900, available from Applied Biosystems, Foster City, California.

The apparatus, system and/or methods described herein can also be used in conjunction with downstream processing of immiscible-fluid-discrete-volumes in conduits.

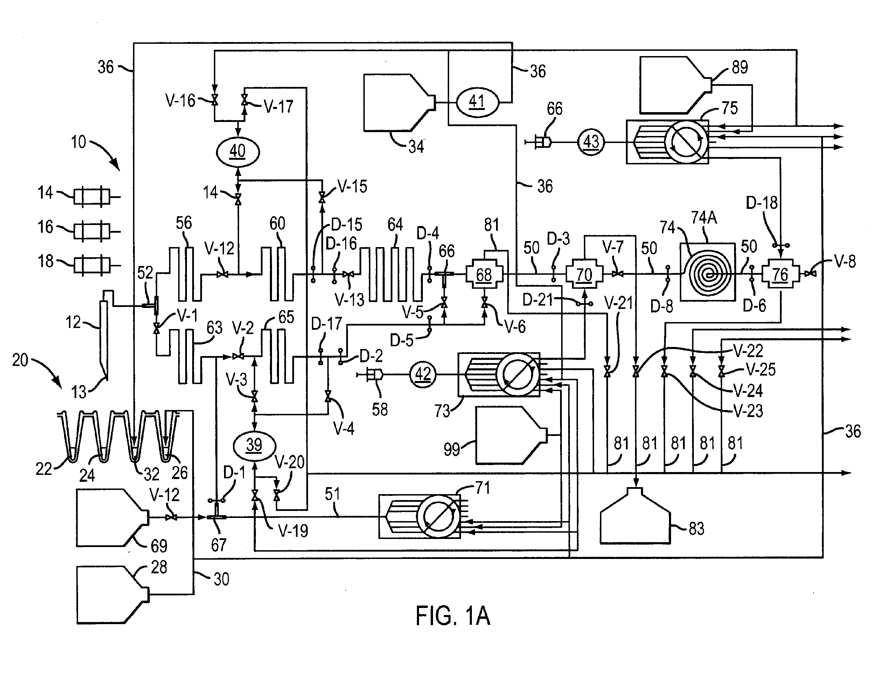

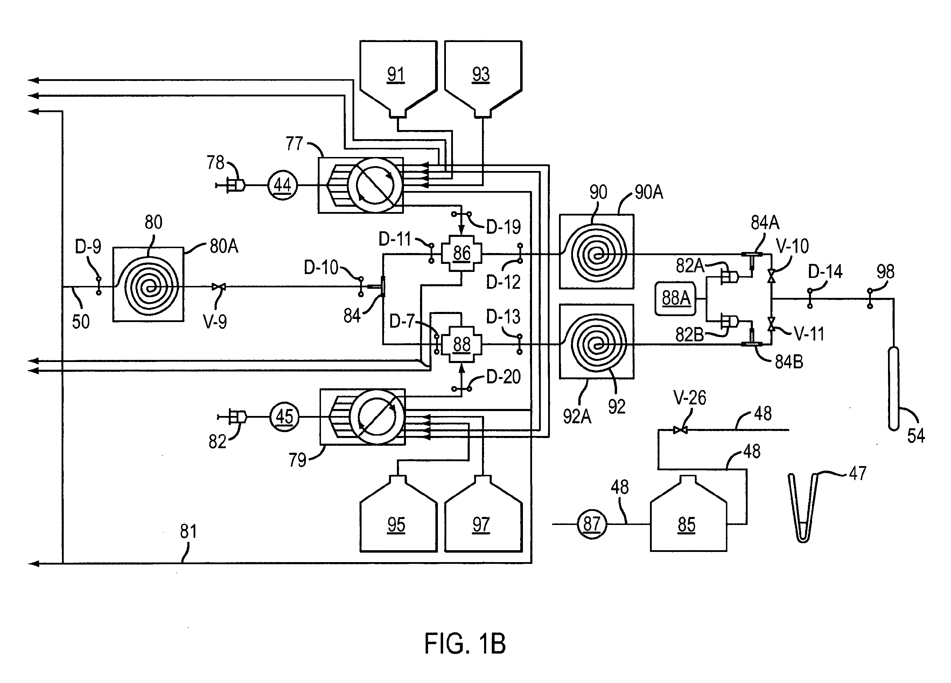

Figs. 1A and 1B are the left-side and right-side, respectively, of a schematic diagram detailing an example of a fluid processing system 10 for processing immiscible-fluid, discrete volumes. The six conduits on the right-hand side of Fig. 1A and terminating in arrow heads pointing to the right are respectively continued as the six conduits shown on the lefthand side of Fig. 1B and terminating in arrow heads pointing to the left, such that the top tube of each respective six depicted are continuations of each other, and so on going down the figures.

Generally, system 10 can be configured to perform different types of assays on fluids introduced thereinto. The amounts and types of fluids introduced into system 10 can be varied depending on a particular assay to be performed. Exemplary assays can include, for example, de novo nucleic acid sequencing reactions, and nucleic acid resequencing reactions, as discussed herein. An exemplary type of sample preparation can be used for genotyping, gene-expression, methylation analysis, and/or directed medical sequencing (VariantSEQr™, for example) that requires multiple liquids to be brought together in an aqueous discrete volume. For example, in a gene-expression application, each aqueous discrete volume can contain individual primer sets. The sample to be analyzed, for example, complementary DNA (cDNA), can be added to each aqueous discrete volume. In the VariantSEQr™ application, for example, an aqueous discrete volume can comprise a primer set, and genomic DNA can be added to that discrete volume

According to various embodiments, one or more samples 22, 24, can be introduced to system 10. Samples 22 and 24, for example, can comprise a nucleic acid containing fluid. According to some embodiments, the nucleic acid contained in a sample can be, for example, a single copy of a genomic DNA sequence of an organism, or complementary DNA from an organism.

In some embodiments, a plurality of fluids can be received by fluid processing system 10 through an immiscible-fluid-discrete-volume-forming tube 12, which is a part of main conduit system 50. Other types of conduits may be used instead of a tube to accept the introduction of fluids into fluid processing system 10. Immiscible-fluid-discrete-volume-forming tube 12 can be part of a system that can comprise, for example, a pump or another apparatus adapted to produce controlled intake of fluids through intake tip 13 into immiscible-fluid-discrete-volume-forming tube 12. Motive force providers for moving fluids into and along a tube or other type of conduit include differential pressure, displacement, electowetting, optoelectrowetting, magnetohydrodynamic "pumps" among others. The immiscible-fluid-discrete-volume-forming conduit 12 can be adapted to control an introduction unit to introduce alternate volumes of aqueous sample fluid and spacing fluid that together form discrete volumes of aqueous sample fluid in contact with spacing fluid, i.e., aqueous sample immiscible-fluid-discrete-volumes, in the at least one conduit wherein each aqueous sample immiscible-fluid-discrete-volume can comprise a maximum outer dimension that is equal to or slightly less than the maximum inner cross-sectional dimension of immiscible-fluid-discrete-volume-forming conduit 12. One of skill in the art will understand that the maximum inner cross-sectional dimension of a tube is the inner diameter of the tube if the tube has a circular cross-section.

According to various embodiments, immiscible-fluid-discrete-volume-forming tube 12 can comprise a tip 13. Tip 13 can interface with fluids to be drawn into system 10. Tip 13 can comprise an angled surface or have any suitable geometry such that the creation of air bubbles in immiscible-fluid-discrete-volume-forming tube 12 is minimized or eliminated when tip 13 contacts and draws in a fluid. Immiscible-fluid-discrete-volume-forming tube 12 can be robotically controlled, or manually controlled. Robotic configurations can comprise, for example, stepper motors 14, 16, and 18, which can move immiscible-fluid-discrete-volume-forming tube 12 in X-axis, Y-axis, and Z-axis directions, respectively. In some embodiments, tube 12 can be moved in the Z-axis direction by a stepper motor 18, and a fluid container can be moved in the X-axis and Y-axis directions by stepper motors 14 and 16, respectively. In some embodiments, tube 12 can be stationary and a fluid container can be moved in the X-axis, Y-axis, and Z-axis directions by stepper motors 14, 16, and 19, respectively. Motive force providers other than stepper motors can be used.

According to various embodiments, a variety of fluids can be introduced into fluid processing system 10, in a number of different combinations, depending on the particular type of assay to be performed. The fluids can reside in or on any suitable fluid retaining device, for example, in the wells of a multi-well plate 20, an opto-electrowetting plate, a tube of preformed slugs, a tube of stable emulsified nanodroplets, individual tubes, strips of tubes, vials, flexible bags or the like.

According to some embodiments, fluid processing system 10 can comprise a number of different fluid conduits and fluid control devices. The following description applies to the embodiment as illustrated in Figs. 1A and 1B, but one skilled in the art will understand that alterations to fluid processing system 10 can be made while the teachings remain within the scope of the present teachings. As illustrated, fluid processing system 10 can comprise a main system conduit 50. Main conduit system 50 can comprise a plurality of tubes connecting together, for example, the following exemplary components: T-junctions 52, 66, and 84; holding conduits 56, 60, 63, 64 and 65; valves V-1, V-2, V-5, V-6, V-7, V-8, V-9, V-10, V-11, V-12, and V-13; cross-junctions 68, 70, 76, 86, and 88; and thermal-spirals 74, 80, 90, and 92. Along conduit 50, thermal spirals 74, 80, 90, and 92 can be in thermal contact with respective thermal cyclers 74A, 80A, 90A, and 92A. Each thermal cycler 74A, 80A, 90A, and 92A can independently comprise a liquid bath, an oven, a plate, a block comprising fluid passages therein, a peltier device, or the like thermal cycling device.

Main conduit system 50 can provide a fluid communication between T-junction 52 and output tube 54. From T-junction 52, conduit system 50 comprises two pathways that intersect at cross-junction 68 and at T 66. A first pathway can take a fluid sequentially through holding tubes 56, 60 and 64, and T-junction 66, before reaching cross-junction 68. A second pathway can take a fluid sequentially through holding tubes 63, and 65, and through either T-junction 66, to cross-junction 68, or directly to cross-junction 68. Both the first pathway and the second pathways are configured to hold fluids for later analysis and are configured to interface with pumps for moving fluids along the conduits as discussed below.

From cross-junction 70, fluids can move sequentially to thermal-spiral 74, cross-junction 76, thermal-spiral 80, and T-junction 84. At T-junction 84 fluids can sequentially move either through cross-junction 86, thermal-spiral 90, and output tube 54, or through cross-junction 88, thermal-spiral 92, and an output tube 54.

According to some embodiments fluid processing system 10 can comprise pumps 39 and 40. Pump 40 can be configured to remove or add oil to main conduit system 50, and thereby move fluids located therein. Pump 39 can be configured to remove or add oil to main conduit system 50 to move fluids located therein. All of the pumps described herein can create positive and/or negative pressures in the various conduits of system 10.

According to various embodiments, a T-junction can comprise any junction having three discrete pathways extending from, for example, either a Y-junction or a T-junction. In various embodiments, the junction can comprise a valve-less junction where a stream of aqueous sample fluid and a stream of non-aqueous spacing fluid can meet and form at least discrete volumes of the aqueous sample fluid in contact with the non-aqueous spacing fluid. For example, microfabrication technology and the application of electrokinetics or magneto hydrodynamics can achieve fluid pumping in valve-less, electronically controlled systems. Components comprising shape-optimized channel turns, optimal injection methods, micromixers, and/or high flow rate electroosmotic pumps can be used in such a valve-less system.

According to some embodiments, system 10 can comprise discrete volume detectors D-1, D-2, D-3, D-4, D-5, D-6, D-7, D-8, D-9, D-10, D-11, D-12, D-13, D-14, D-15, D-16, D-17, D-18, D-19, and D-20, and detector 98. The discrete-volume detectors can comprise, for example, fluorescent or infra-red, refractive-index detectors, and possibly capacitive and absorption detectors. In Figs. 1A and 1B, all of the detectors depicted are infrared detectors with the exception of detector 98 which is a fluorescent signal detector, although other arrangements can be used. The discrete volume detectors can be configured to distinguish immiscible-fluid, discrete volumes from spacing fluid or oil as the discrete volumes travel through the conduits of system 10.

According to various embodiments, the system can comprise a thermal-cycling device or thermal cycler, adapted to thermally cycle an aqueous immiscible-fluid, discrete volume in a conduit disposed thereon or therein. In some embodiments, the conduit can contact the thermal cycler in a single straight-line segment, or a coil around the external perimeter of thermal cycler, or a spiral of decreasing radius on one surface, or a serpentine pattern across one or more surfaces of thermal cycler. The thermal-cycling device can comprise a heat source, for example, a radiant heat source, a non-radiant heat source, a peltier device, or the like, and a cooling source, for example, a fan, an air jet, or a liquid-circulating system in a thermal block. The thermal-cycling device can comprise one or more temperature sensors and one or more control units for controlling heating and cooling according to a desired or programmed thermal cycle.

The conduits of the present teachings can comprise capillary tubes having an inner diameter and the inner diameter can be, for example, about 1000 microns or less, for example, about 800 microns or less, or about 500 microns or less. In some embodiments, the tube has a minimum inner dimension, or diameter, of from about 1.0 micron to about 100 microns, or from about 50 microns to about 75 microns. In other embodiments, the tube can have an inner diameter greater than about 300 microns. In some embodiments, main conduit system 50 can comprise tubing with a 635 micron inner diameter. In some embodiments, thermal spirals after T-84 can comprise tubing with a 480 micron inner diameter. In some embodiments conduit system 50 can comprise tubing with an inner diameter in a range of from about 380 microns to about 635 microns, with the smallest diameters at the ends of the system. Other details about the thermal-cycling device, capillary channel, and other system components will become apparent in view of the teachings herein.

System 10 can comprise an single molecule amplification fluid ("SMAF") conduit system 51. SMAF tube system 51 can supply sample fluid to a T-junction through positive pressure rather than by aspiration. SMAF conduit system 51 can comprise a supply conduit connected to and in fluid communication with a supply of single molecule amplification fluid. The SMAF can comprise a solution or mixture of target nucleic acids diluted to a degree such that there is an average of less than about one target nucleic acid per volume of single molecule amplification fluid that is used to make an immiscible-fluid-discrete-volume. An exemplary concentration of target molecules can be 0.4 molecule per volume used to make an immiscible-fluid-discrete-volume. SMAF conduit system 51 can comprise conduits connecting a SMAF reservoir 69 sequentially to valve V-18 and T-junction 67. SAMF conduit system 51 can comprise conduits that connect T-junction 67 to main conduit system 50 and a rotary valve 71.

Fluid processing system 10 can comprise rotary valves 71, 73, 75, 77, and 79. Each rotary valve can function to direct the flow of metered amounts of different reagents from different respective reagent reservoirs connected thereto, as described below, to main conduit system 50. Syringe pumps 58, 66, 78, and 82 can be in fluid communication with rotary valves 73, 75, 77 and 79, respectively. Pumps 42, 43, 44, and 45 can be in fluid communication with rotary valves 73, 75, 77 and 79, respectively.

Fluid processing system 10 can comprise a first waste tube system 81. Waste tube system 81 can comprise conduits connecting the following components: valves V-17, V-20, V-21, V-22, V-23, V-24, V-25; and a waste reservoir 83. Waste tube system 81 can provide a fluid communication between and cross-junctions 68, 70, 76, 86, and 88 and waste reservoir 83.

Fluid processing system 10 can comprise a second waste tube system 48. Second waste tube system 48 can comprise conduits connecting a pump 87, a waste reservoir 85, and a valve V-26, that interface with output tube 54. Second waste tube system 48 can be used to remove liquids from output tube 54.

Fluid processing system 10 can comprise reagent reservoirs 89, 91, 93, 95, 97, and 99, which can be in fluid communication with rotary valves 75, 77, 77, 79, 79, and 73, respectively. Reagent reservoir 89 can contain, for example, an exo-nuclease and shrimp alkaline phosphatase. Reagent reservoir 91 can contain, for example, nucleic acid amplification reaction forward primers. Reagent reservoir 93 can contain, for example, nucleic acid amplification reaction chain terminating dyes. Reagent reservoir 95 can contain, for example, nucleic acid amplification reaction reverse primers. Reagent reservoir 97 can contain, for example, nucleic acid amplification reaction chain terminating dyes. Reagent reservoir 99 can contain, for example, a nucleic acid amplification reaction master mix comprising, for example, reactive single base nucleotides, buffer, a polymerase, and the like, for example, to carry out a polymerase chain reaction.

According to various embodiments, fluid processing system 10 can comprise a rinse system 30. Rinse system 30 can provide a fluid communication between a rinse fluid reservoir 28, rotary valve 73, rotary valve 75, rotary valve 77, rotary valve 79, and immiscible-fluid-discrete-volume-forming tube 12. Rinse fluid reservoir 28 can contain a rinse fluid 26. Rinse fluid 26 can comprise microbiologic grade water, for example, distilled, de-ionized water.

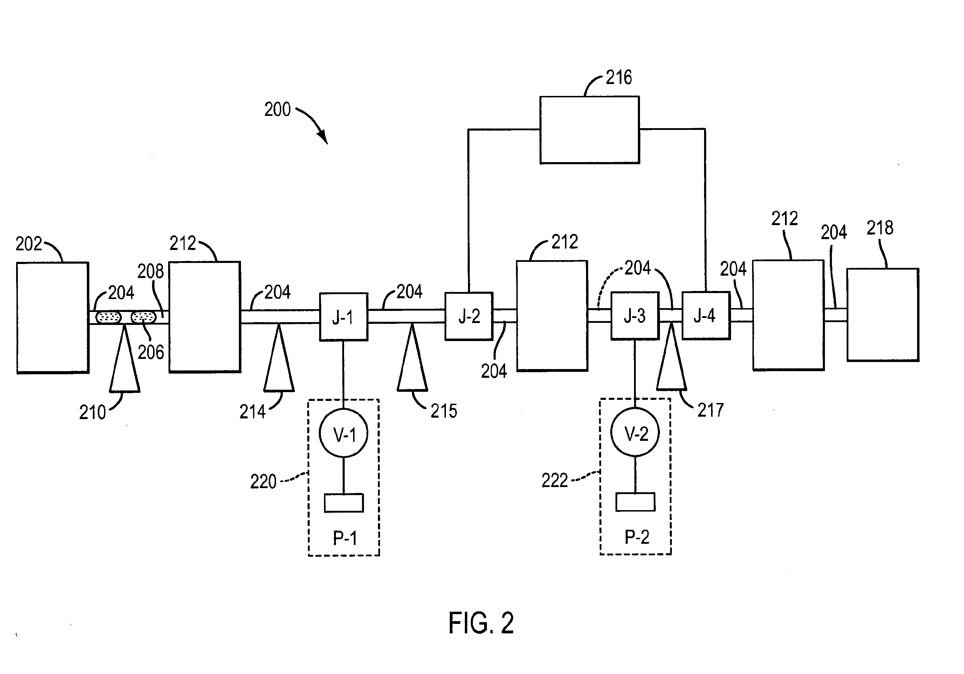

Rinse fluid 26 can be used to remove residual sample, for example, from immiscible-fluid-discrete-volume-forming tube 12. Rinse fluid can be provided to multi-well plate 20, by way of rinse tube system 30. In some embodiments, rinse fluid 26 can be added to immiscible-fluid, discrete volumes to adjust the volume or concentration thereof, in conjunction with an addition station, as described in FIG. 2.

According to various embodiments, fluid processing system 10 can comprise a spacing fluid tube system 36. Spacing fluid tube system 36 can provide a fluid communication between a spacing fluid reservoir 34, vacuum pump 41, and multi-well plate 20. Spacing fluid reservoir 34 can contain an oil 32 or other spacing fluid that is immiscible with an immiscible-fluid-discrete-volume-forming fluid, for example, an aqueous slug fluid.

In some embodiments, the spacing fluid can be non-aqueous. The spacing fluid can comprise an organic phase, for example, a polydimethylsiloxane oil, a mineral oil (e.g., a light white mineral oil), a silicon oil, a hydrocarbon oil (e.g., decane), a fluorinated fluid or a combination thereof.

Fluorinated compounds such as, for example, perfluoroooctyl bromide, perfluorodecalin, perfluoro-1,2-dimethylcyclohexane, FC 87, FC 72, FC 84, FC 77, FC 3255, FC 3283, FC 40, FC 43, FC 70, FC 5312 (all "FC" compounds are available from 3M, St. Paul, Minnesota), the Novec® line of HFE compounds (also available from 3M, St. Paul, Minnesota), such as, for example, HFE-7000, HFE-7100, HFE-7200, HFE-7500, and perfluorooctylethane can also be used as the spacing fluid. Combinations, mixtures, and solutions of the above materials can also be used as the spacing fluid.

In some embodiments, fluorinated alcohols, such as, for example, 1H, 1H, 2H, 2H-perfluoro-decan-1-ol, 1H, 1H, 2H, 2H-perfluoro-octan-1-ol, and 1H, 1H-perfluoro-1-nonanol can be added to a fluorinated compound, such as those listed above, to improve the stability of aqueous discrete volumes within the spacing fluid, but still maintain the ability to coalesce upon contact. In some embodiments, fluorinated alcohols can be added in a range of approximately 0.1% to approximately 5% by weight. In some embodiments, the fluorinated alcohol additive can be approximately 0.1%, 0.2%, 0.5%, 1.0%, 1.5%, 2.0%, 3.0%, 4.0% or 5% by weight of the fluorinated compound. In some embodiments, the fluorinated alcohol additive can be from approximately 1% to approximately 10% by volume of the fluorinated compound. In some embodiments, the fluorinated alcohol additive may comprise approximately 1%, 2%, 3%, 4%, 5%, 6%, 7%, 8%, 9%, or 10% by volume of the spacing fluid. In some embodiments, F-alkyl dimorpholinophosphates can be added as surfactants to fluorinated compounds.

In some embodiments, the organic phase can include non ionic surfactants such as sorbitan monooleate (Span 80 (no. S-6760, Sigma)), polyoxyethylenesorbitan monooleate (Tween 80 (no. S-8074, Sigma)), sorbitan monostearate (Span 60), octylphenoxyethoxyethanol (Triton X-100 (no. T9284, Sigma)). In some embodiments, Span 80 can be added in an amount ranging from about 1.0% to about 5.0%, or about 3.0% to about 4.5%. In some embodiments, adding surfactants in the quantities of 4.5% Span 80, 0.40% Tween 80, and 0.05% Triton X-100 to mineral (no. M-3516, Sigma) can result in the creation of stable emulsified droplets.

In some embodiments, the organic phase can include ionic surfactants, such as sodium deoxycholate, sodium cholate, and sodium taurocholate. In some embodiments, the organic phase can include chemically inert silicone-based surfactants, such as, for example, polysiloxane-polycetyl-polyethylene glycol copolymer.

In some embodiments, the non-aqueous, spacing fluid can have a viscosity between approximately 0.5 to approximately 0.75 centistokes. In some embodiments, the non-aqueous spacing fluid can have a viscosity between approximately 0.75 centistokes to about 2.0 centistokes. In some embodiments, the non-aqueous spacing fluid can have a viscosity greater than 2.0 centistokes. In some embodiments, the non-aqueous spacing fluid can have a viscosity between 0.5 to greater than about 2.0 centistokes. In some embodiments, the non-aqueous spacing fluid can have a viscosity greater than 2.0 centistokes. In some embodiments, the non-aqueous, spacing fluid can have a boiling point greater than or equal to 100 C.

Spacing fluid 32 can function to separate discrete volumes of an immiscible-fluid-discrete-volume-forming fluid, for example, an aqueous sample fluid, before, during, or after the immiscible-fluid-discrete-volume-forming fluid has been introduced into system 10. Spacing fluid can be provided to multi-well plate 20, from a spacing fluid reservoir 34, by way of a spacing fluid tube system 36.

According to some embodiments, a de novo nucleic acid sequencing method is provided that uses system 10. The de novo sequencing method can be used to sequence an entire genome or portions thereof. The de novo sequencing method can be especially useful when the sequence of the organism is unknown.

In some embodiments, a de novo sequencing method comprises pre-processing a sample, separating the sample into a set of immiscible-fluid, discrete volumes, optionally adding amplification reagents to each discrete volume of the set, amplifying nucleic acids in the set of immiscible-fluid, discrete volumes to form a set of amplified immiscible-fluid, discrete volumes, optionally detecting, and removing, discrete volumes without amplified sample molecules therein, adding primer and dNTP deactivation agents to each discrete volume in the set, or optionally, to only those with amplified sample molecules, incubating the set of amplified immiscible-fluid, discrete volumes with primer and dNTP deactivation agents, subjecting the resulting nucleic acids to sequencing conditions to form detectable products, and detecting the detectable products.

In some embodiments, the method can comprise pre-processing a sample before it is input into system 10. The pre-processing of a sample can comprise fragmenting the nucleic acid present in the sample. The fragmentation can be accomplished by any suitable method known in the art. For example, the nucleic acid can be fragmented by enzymatic digestion, or physical disruption methods, for example, hydro-shearing or sonication. In some embodiments the nucleic acid can be fragmented to an average size of about 500 B, 750 B, 850 B, 1 KB, 2 KB, or 3 KB, for example.

According to some embodiments, the pre-processing of sample can comprise ligating sequences to a sample. Universal sequences can be used to facilitate universal nucleic acid amplification. Universal sequences can be artificial sequences that generally have no homology with the target nucleic acids. Universal sequences can be designed to resist the formation of dimers between themselves. Universal sequences can be designed to bind with analogous primers with a consistent efficiency.

According to some embodiments, the present teachings can encompass a de novo sequencing method wherein universal sequences can be ligated to the 5' and 3' ends of the DNA fragments in a sample by, for example, T-4 DNA ligase, thereby forming a universal tail. The universal tail sequences can function as sites of complementarity for zip code primers. Details of universal tail procedures can be found in U.S. Pat. App. No. 2004/0185484, to Costa et al.

According to various embodiments, the amplifying of a nucleic acid can comprise a thermal cycling nucleic acid sequence amplification process or an isothermal nucleic acid sequence amplification process. If a thermal cycling nucleic acid sequence amplification process is used, the process can comprise, for example, a polymerase chain reaction (PCR). The nucleic acid sequence amplification reaction can comprise an exponential amplification process, for example, PCR, or a linear amplification process, as can occur during, for example, during Sanger cycle sequencing. In various embodiments, other nucleic acid amplification processes can be used, for example, ligase chain reaction (PCR), nucleic acid sequence based amplification (NASBA), Q-beta replicase (QB) amplification, or strand displacement amplification (SDA). These alternatives, as well as others known to one skilled in the art, can be used either by themselves or in combination with PCR to amplify nucleic acids.

According to various embodiments, nucleic acid sequence processing methods comprising a first type of nucleic acid amplification reaction followed by one or more of a second different type of amplification reaction, and/or detection assay reaction, can be carried out.

According to some embodiments, the present teaching can comprise a method of de novo sequencing wherein pre-processing of sample can comprise adding zip code primers to a sample of nucleic acid having universal tail sequences ligated therein. Zip code primers can be complementary to the universal tail sequences. The use of zip code tails sequences and zip code primers can reduce the need for target specific primers, resulting in significant cost savings as well as greater assay flexibility.

According to various embodiments, pre-processing a sample can comprise adding to the sample reactants to facilitate a nucleic acid amplification reaction. For example, the four dNTP's (dATP, dTTP, dGTP, and dCTP), a polymerase, oligonucleotide primers, and/or chelating. agents can be added to the sample. Oligonucleotide primers can be chosen as determined by one of skill in the art to accomplish the desired objective, for example, universal primers can be used.

According to various embodiments, pre-processing a sample can comprise diluting the sample with a miscible solvent, vehicle, or carrier. The sample can be diluted at a ratio of 1:1, 1:10, 1:100, 1:1000, or 1:10,000, for example. Exemplary ranges of dilution can be from about 1:1 to about 1:100, or from about 1:10 to about 1:50. For example, the sample can be diluted such that only a single fragment of nucleic acid is present per 500 nanoliters of diluted sample, or per 200 nanoliters of diluted sample. In some embodiments, the concentration of target fragments can be based on the size of the immiscible-fluid-discrete-volumes generated that carry the target fragments, such that an average of about 1 target fragment is present per 1.4 immiscible-fluid-discrete-volumes generated. According to various embodiments, the sample can be diluted such that at least 50% immiscible-fluid-discrete-volumes produced from a sample in the process described below can each comprise a single target nucleic acid sequence. In various other embodiments, less than about 50% of the immiscible-fluid-discrete-volumes produced can each comprise a single target nucleic acid sequence. In other embodiments, at least 1% or more, 5% or more, 10% or more, or 20% or more can comprise a single target nucleic acid sequence, for example, from about 10% to about 50% or from about 20% to about 40%.

After optional preprocessing, the sample fluid is introduced to system 10 to form one or more discrete volumes of the sample fluid in a spacing fluid with which it is immiscible. A plurality of immiscible-fluid, discrete volumes can be associated together as a set of immiscible-fluid, discrete volumes. Each set of immiscible-fluid, discrete volumes can comprise immiscible-fluid, discrete volumes separated from one another by a spacing fluid, for example, an oil. Each immiscible-fluid-discrete-volume of a set can be equally spaced from one or more adjacent immiscible-fluid, discrete volumes of the set. Multiple sets of immiscible-fluid, discrete volumes can be present at the same time in main conduit 50. Each set of immiscible-fluid, discrete volumes can be separated from one or more other sets of immiscible-fluid, discrete volumes by larger volumes of spacing fluid, which in a tube of constant cross-sectional area size and shape is visible by greater length between the trailing end of the last discrete volume of the upstream set and the leading end of the first discrete volume in the downstream set. In some embodiments, two or more sets of immiscible-fluid, discrete volumes are spaced from one another a distance that is greater than the average distance between adjacent immiscible-fluid, discrete volumes with the same set.

In the embodiment depicted in Figs. 1A and 1B, immiscible-fluid, discrete volumes that have been aspirated into immiscible-fluid-discrete-volume-forming tube 12 can be moved into holding tube 56 by suction produced by vacuum pump 40. In some embodiments, pump 40 can be a syringe pump.

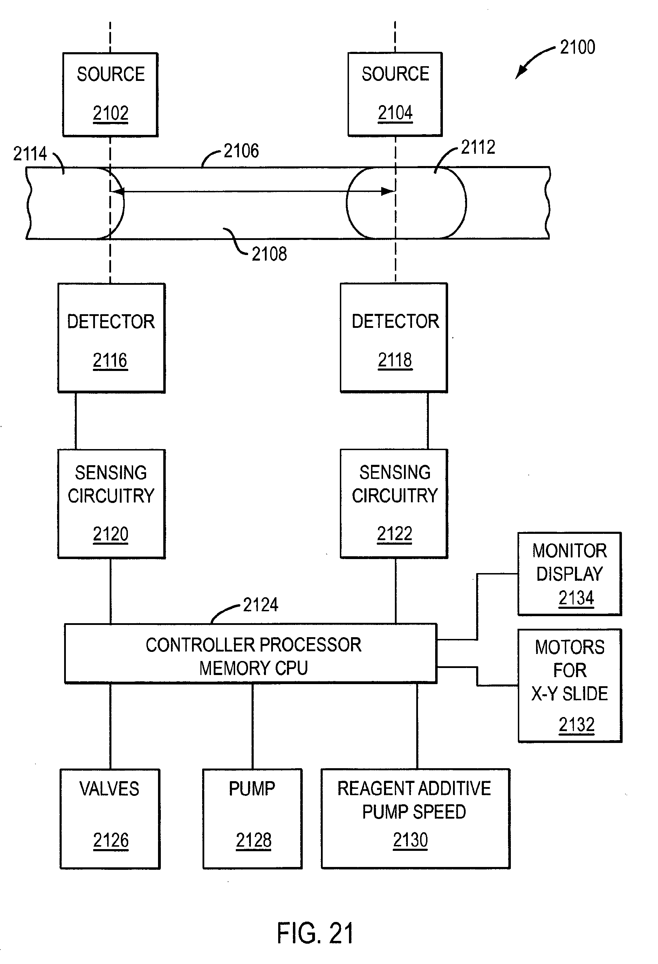

According to some embodiments, the method can comprise moving a set of immiscible-fluid, discrete volumes, from T-junction 52, to cross-junction 70, by way of conduit system 50. If a set of immiscible-fluid, discrete volumes does not contain nucleic acid amplification reactants, the reactants can be added to each immiscible-fluid-discrete-volume of the set of immiscible-fluid, discrete volumes at cross-junction 70. More details about exemplary methods of adding additional miscible liquid to immiscible-fluid, discrete volumes is provided herein, at least in FIGS. 2, 21-28, and 29A-C. As illustrated in FIGS. 1A and 1B, reactant addition to each immiscible-fluid-discrete-volume can be metered by rotary valves 71 and 73. Detector D-3 can detect the arrival of the beginning and/or the end of a set of sample immiscible-fluid, discrete volumes at cross-junction 70. More details about an exemplary method of detecting the size and speed of immiscible-fluid, discrete volumes is provided herein, at least in FIGS. 21 - 22. Detector D-21 can detect the arrival of the beginning and/or the end of immiscible-fluid, discrete volumes at cross-junction 70. In some embodiments, valve V-7 can control the movement of a set of immiscible-fluid, discrete volumes out of cross-junction 70. In some embodiments, valve V7 can isolate a thermal cycle section during thermal cycling.

According to some embodiments, the method can comprise moving a set of immiscible-fluid, discrete volumes from cross-junction 70, through main conduit system 50, to thermal-spiral 74. Detector D-8 can be used to detect the arrival of a set of immiscible-fluid, discrete volumes at thermal-spiral 74. Detector D-8 can be used to detect the end of a set of immiscible-fluid, discrete volumes, and thereby detect that a set of immiscible-fluid, discrete volumes is disposed in thermal-spiral 74. A set of immiscible-fluid, discrete volumes can be thermally cycled, for one or more cycles, for example, for from about 5 to about 50 temperature cycles or from about 20 to about 30 temperature cycles.

According to various embodiments, the method can comprise introducing polymerase chain reaction inactivating reagents into main tube 50 after amplifying the at least one target nucleic acid sequence and before subjecting the nucleic acid sequence to a sequencing reaction. The reagents can be used to inactivate or remove or eliminate excess primers and/or dNTP's. The inactivating reagents can be introduced at an junction in the capillary channel, for example, after an immiscible-fluid-discrete-volume to be inactivated is aligned with the junction. The junction can comprise, for example, a T-junction.

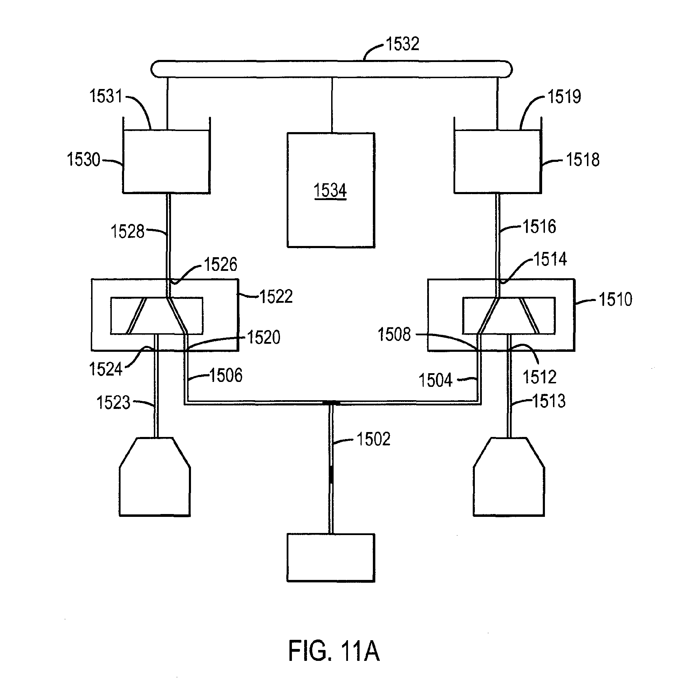

According to some embodiments the method can comprise moving a set of immiscible-fluid, discrete volumes from thermal-spiral 74, through cross-junction 76. As the set of immiscible-fluid, discrete volumes moves through cross-junction 76, the method can comprise adding exonuclease and shrimp alkaline phosphatase to each immiscible-fluid-discrete-volume of the set of immiscible-fluid, discrete volumes. For example, the exonuclease and shrimp alkaline phosphatase "SAP" can be metered out in discrete volumes which merge respectively with the immiscible-fluid, discrete volumes of a set of immiscible-fluid, discrete volumes at an junction in rotary valve 77. More details about metering "addition" liquid, such as, for example, exonuclease and SAP is provided herein, at least in FIGS. 11A-B, 21-24, and 25-27 and their corresponding descriptions. For example, exonuclease and shrimp alkaline phosphatase can be added to each immiscible-fluid-discrete-volume of the set of immiscible-fluid, discrete volumes in cross-junction 76.

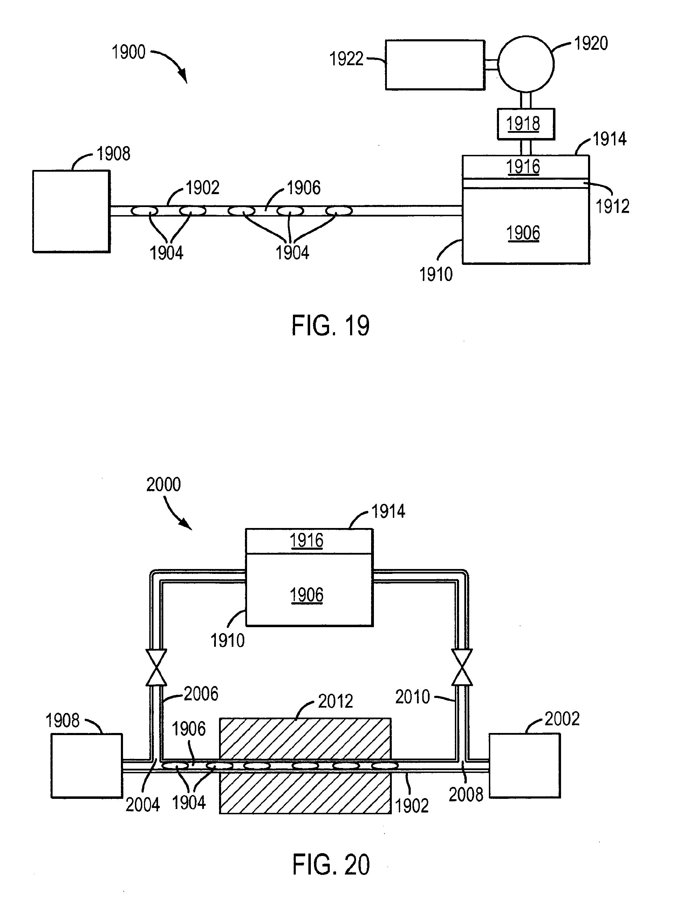

In the exemplary system shown, detector D-6 can detect the arrival of the beginning and/or the end of a set of sample discrete volumes at cross-junction 76. Detector D-18 can detect the arrival of the beginning and/or the end of one or more immiscible-fluid, discrete volumes of exonuclease and shrimp alkaline phosphatase at cross-junction 76. Valve V-8 can control the movement of a set of immiscible-fluid, discrete volumes out of cross-junction 76. In some embodiments, V8 can be used to isolate one or more thermal spirals from each other. More details about an exemplary method of isolating a thermal spiral is provided herein, at least in FIGS. 19-20, and the corresponding description.

In the exemplary embodiment shown, a set of immiscible-fluid, discrete volumes containing exonuclease and shrimp alkaline phosphatase can be moved into thermal-spiral 80, via main conduit system 50. Detector D-9 can detect the arrival of the beginning and/or the end of a set of immiscible-fluid, discrete volumes at thermal-spiral 80. The set of immiscible-fluid, discrete volumes can be incubated at from about 25°C to about 35°C for a time period of from about one minute, to about 60 minutes or from about two minutes to about 10 minutes. The incubation step can function to facilitate the activities of the exonuclease and shrimp alkaline phosphatase. A set of immiscible-fluid, discrete volumes can be further incubated at a temperature of from about 75°C to about 85°C, for a time period of from about 10 seconds to about 10 minutes, or from about one minute to about five minutes. The incubation at from about 75°C to about 85°C can function to heat-kill any enzymes that might still be present in the set of immiscible-fluid, discrete volumes.

According to some embodiments, the method can comprise moving a set of immiscible-fluid-discrete-volumes to T-junction 84. Valve V-9 can control the movement of a set of immiscible-fluid-discrete-volumes from thermal spiral 80, to T-junction 84. Detector D-10 can detect the arrival of the beginning and/or the end of a set of immiscible-fluid-discrete-volumes at T-junction 84. The method can comprise dividing one or more immiscible-fluid, discrete volumes of a set of immiscible-fluid discrete volumes into two or more smaller immiscible-fluid-discrete volumes to form two newly formed sets of equal number of immiscible-fluid discrete volumes, but containing immiscible-fluid discrete volumes of smaller volume. More details about an exemplary method of splitting immiscible-fluid, discrete volumes into smaller immiscible-fluid, discrete volumes is provided herein, at least in FIGS. 11A-B, and 12-16 and the corresponding description. The method can comprise moving one newly created set of immiscible-fluid, discrete volumes along main conduit system 50, to cross-intersection 86. Forward primers and chain terminating dyes can be moved from reservoirs 91 and 93, to rotary valve 77. The forward primers and chain terminating dyes can be metered out by rotary valve 77. The forward primers and chain terminating dyes can be moved to cross-intersection 86 and be added to each immiscible-fluid-discrete-volume of the newly-created set of immiscible-fluid, discrete volumes, thereby creating a forward set of immiscible-fluid, discrete volumes. According to various embodiments, the method can comprise moving the second newly created set of immiscible-fluid, discrete volumes along main conduit system 50, to cross-intersection 88. Reverse primers and chain terminating dyes can be moved from reservoirs 95 and 97, to rotary valve 79. The reverse primers and chain terminating dyes can be metered out by rotary valve 79. The reverse primers and chain terminating dyes reagent can be moved to cross-intersection 86 and be joined with each immiscible-fluid-discrete-volume of the second newly-created set of immiscible-fluid, discrete volumes, thereby creating a reverse set of immiscible-fluid, discrete volumes..

In some embodiments, the method can comprise moving the forward set of immiscible-fluid-discrete-volumes from cross-junction 86, along main conduit system 50, to thermal spiral 90. The forward set of immiscible-fluid-discrete-volumes can be thermally cycled for from about 5 to about 50, temperature cycles, for example, from about 20 to about 40 thermal cycles.

In some embodiments, the method can comprise moving the reverse set of immiscible-fluid-discrete-volumes from cross-junction 88, along main conduit system 50, to thermal spiral 92. The reverse set of immiscible-fluid-discrete-volumes can be thermally cycled for from about 5 to about 50 thermal cycles, for example, from about 20 to about 40 cycles, temperature cycles.

In some embodiments, the method can comprise thermal cycling at least four different sets of immiscible-fluid-discrete-volumes, one in each of thermal spirals 74, 80, 90, and 92. In some embodiments, valves, such as, for example, can be used to isolate each thermal spiral from all other thermal spirals. In some embodiments, the different thermal spirals operate at the same time, but with different thermal profiles. In some embodiments, isolating the thermal expansion and contraction of the fluid in the different spirals can be desirable. In some embodiments, the total time for more than one temperature cycling can be the same. In embodiments where different length thermal cycles are desired, an additional step at non-process inducing temperature can be added for the remainder of the longest thermal cycles.

According to various embodiments, the method can comprise moving the forward and the reverse sets of immiscible-fluid-discrete-volumes from their respective thermal spiral to output conduit 54. Movement can be caused by syringe pumps 82A and 82B that can be controlled independently, or together, by a motor 88A operatively connected thereto. Syringe pumps 82A and 82B can push and pull fluids through respective T-junctions 84A and 84B. This arrangement is useful as syringe pumps 82A and 82B can initially pull immiscible-fluid-discrete-volumes into place in the respective thermal spirals 90 and 92, in conjunction with the positive pressure from the pumps on the upstream side of tee 84. Valves V-10 and V-11 can be switched so that immiscible-fluid-discrete-volumes can be pushed out of system 10. In some embodiments, the pushing can be done with one of pumps 82A and 82B at a time; therefore, there is no need to merge two separate sets of immiscible-fluid-discrete-volumes back together into a single set, but rather the separate sets can be individually dispensed. Output conduit 54 can deposit both sets of immiscible-fluid-discrete-volumes on, for example, a multi-well plate.

According to some embodiments, a dye can be added to one or more immiscible-fluid, discrete volumes of a set of immiscible-fluid, discrete volumes. The dye can comprise a double-strand (ds), nucleic acid intercalating dye, for example, SYBR green, SYBR gold, EVA green, LC green, or the like. The dye can be added to an aqueous immiscible-fluid-discrete-volume-forming fluid, such as an aqueous sample, before it is added to system 10. The dye can be added to a set of immiscible-fluid, discrete volumes at any cross-junction of system 10. The dye can be used to discriminate between immiscible-fluid, discrete volumes that contain ds nucleic acids and immiscible-fluid, discrete volumes that do not contain ds nucleic acids. The immiscible-fluid, discrete volumes that do not contain ds nucleic acids can be removed from output tube 54 before the immiscible-fluid, discrete volumes are deposited on a multi-well plate 47. The immiscible-fluid, discrete volumes that do not contain ds nucleic acids can be moved through second waste tube system 48, to waste reservoir 85. In some embodiments, a dye can be detected by detector 98 to determine whether a discrete volume should be sent to second waste reservoir 85 or be collected. Pump 87 can apply a negative pressure to waste tube system 48, which can cause the movement of immiscible-fluid, discrete volumes into waste reservoir.

Immiscible-fluid, discrete volumes deposited on multi-well plate 47 can be subjected to a sequencing reaction to form a detectable product, and the method of the present teachings can comprise detecting the detectable product. In various embodiments, the detectable product can be detected using, for example, a flow cell or a capillary electrophoresis sequencer. In various other embodiments, an off-capillary detector can be used as deemed appropriate.

Shown below is a table showing a state diagram of various settings that can be implemented for the various valves and detectors of the system shown in Figs. 1A and 1B, to achieve various different functions, for example, to carry out various different immiscible-fluid-discrete-volume processing useful in the above described de novo sequencing method.

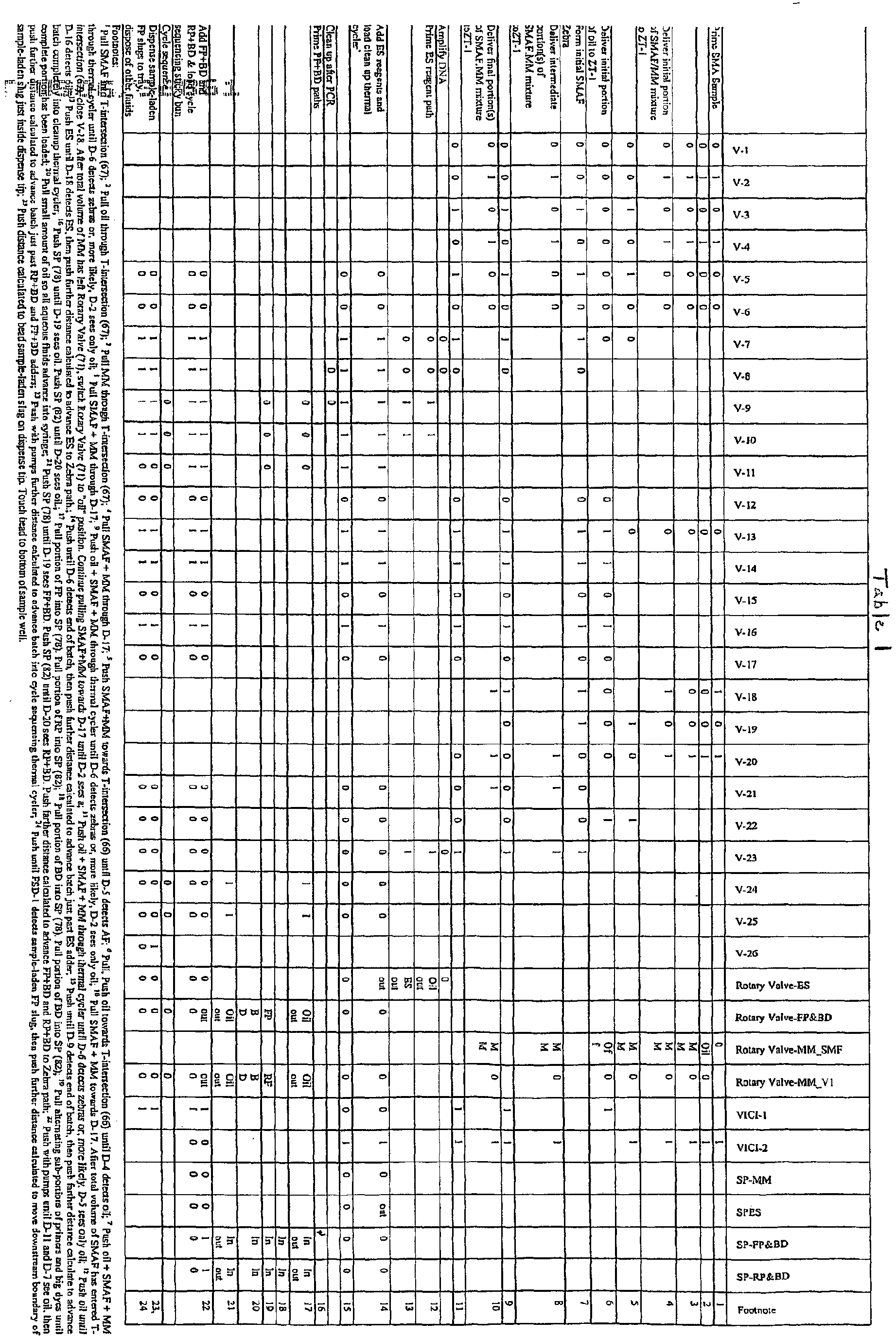

According to various embodiments, the present teachings can encompass a resequencing method using system 10. In general, the resequencing method is similar to the de-novo method described herein with modifications as discussed herein.

In some embodiments, the pre-processing of a sample for resequencing comprises shearing a robust sample of nucleic acid having a plurality of copies of one or more nucleic acids of interest, herein also referred to as target sequences. The nucleic acids in the sample can be sheared. The method can comprise adding a set of gene specific primers, for example, at cross-junction 10, to a set of immiscible-fluid, discrete volumes generated from the sample. Immiscible-fluid, discrete volumes made from the sample can contain a single copy of a nucleic acid fragment or can contain a plurality of copies of one or more different nucleic acid fragments. Each immiscible-fluid-discrete-volume can contain, for example, from about 50 to about 150 or more; 384 or a thousand, or several thousands, or fewer.

In some embodiments, the method can comprise moving a set of immiscible-fluid, discrete volumes comprising the concentrations of primers discussed above, to thermal-spiral 74. The set of immiscible-fluid, discrete volumes can be thermally cycled and thereafter processed in any of the many manners disclosed herein for the de novo sequencing method.

Shown below are Tables 2A and 2B which are the first and second halves of another state diagram of various settings that can be implemented for the various valves and detectors of the system shown in FIGS. 1A and 1B, to achieve various different functions. The various functions can include carrying out various different immiscible-fluid-discrete-volume processing.

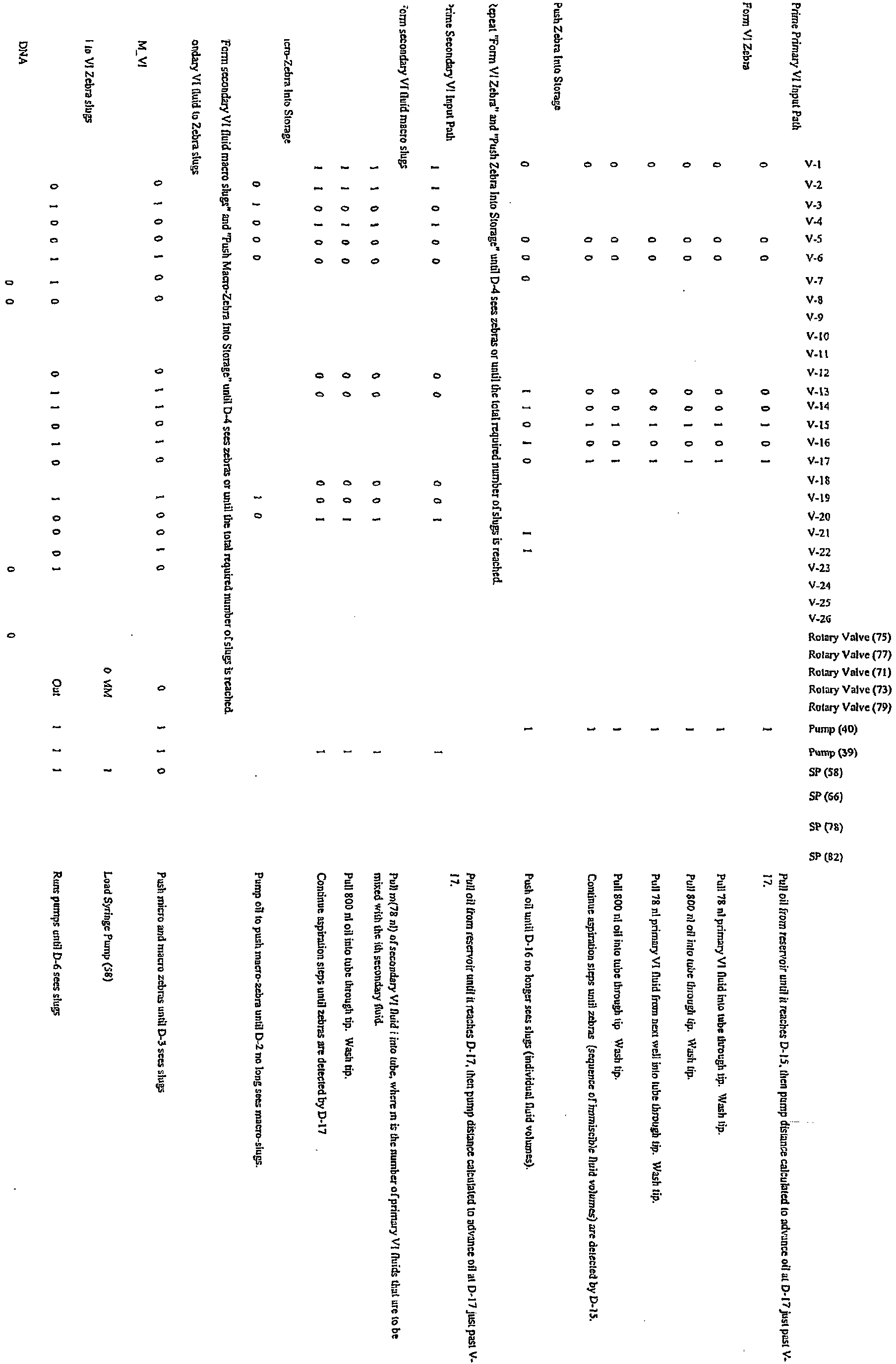

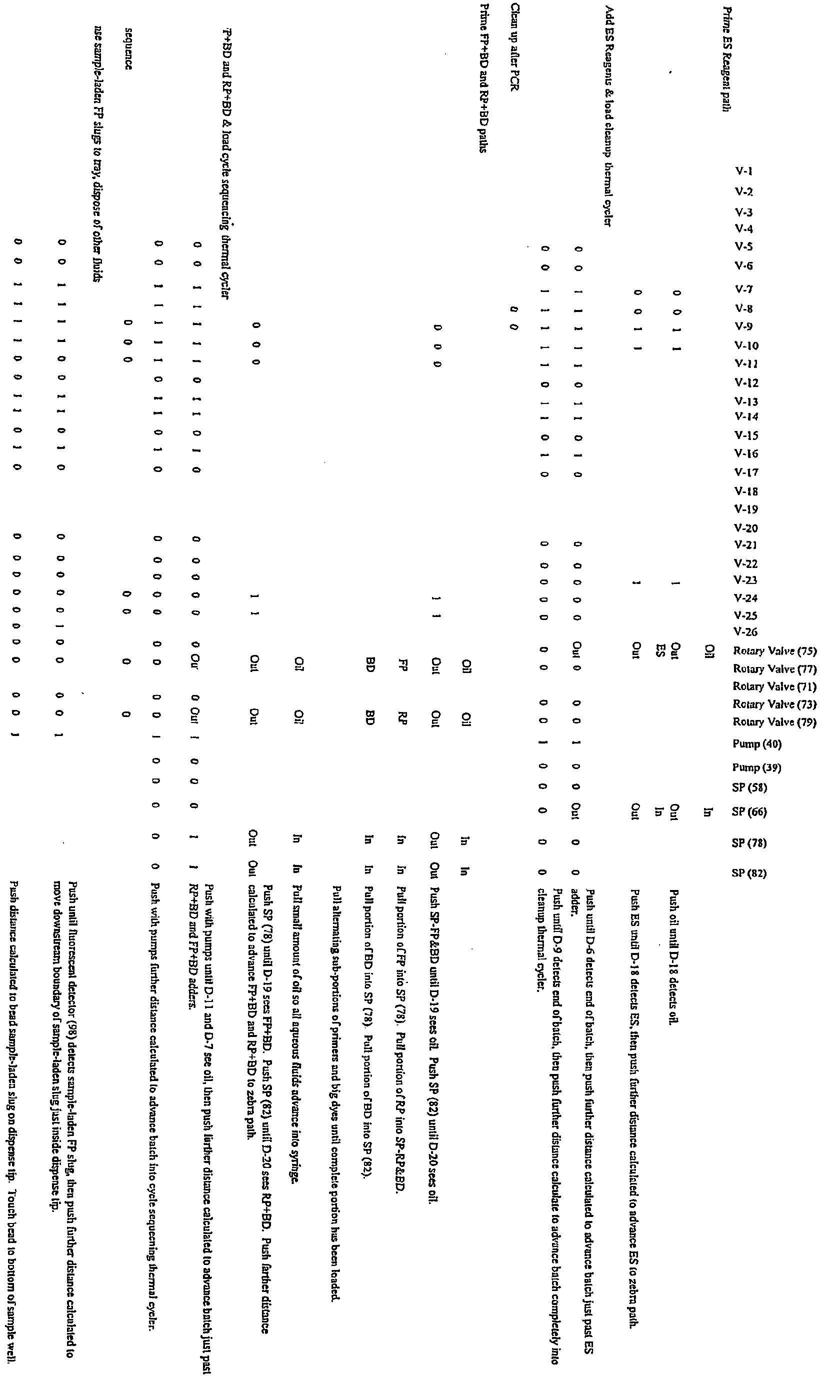

A simplified system 200 is illustrated in FIG. 2. As illustrated, box 202 represents a structure that delivers to tube 204 of system 200 discrete volumes 206 of aqueous liquid in a non-aqueous liquid 208 with which they are immiscible. In some embodiments, such a structure could be a tube of preformed discrete volumes 206 of aqueous fluid. In some embodiments, such a structure could be a chip or other substrate with a channel therein containing the discrete volumes 206 of aqueous fluid. As illustrated, tube 204 extends throughout system 200. After entering tube 204, desired information about aqueous volumes 206 are determined and optionally manipulated by structures in triangle 210. For example, the length and speed of a slug and the distance between two adjacent slugs can be desired information. In that example, a slug detection system can provide that information. If the distance between adjacent slugs does not meet preferred values, then additional spacing fluid can be added between the trailing point of the first slug and the leading point of the second slug, or one of the slugs could be held in an electric field, for example, to allow more of the existing spacing fluid to flow past it in tube 203. If the length, and therefore the volume, of an aqueous discrete volume does not meet preferred values, additional non-reactive, miscible liquid can be added by an apparatus at that area of tube 204. Triangle 210 represents these and other structures of discrete volume characteristic detection and manipulation. Examples of these structures and/or component parts of thereof are described herein.

System 200, as illustrated in FIG. 2, next incorporates a processing section 212 of tube 204 (not illustrated, but in the box), which can include, for example, vibration, heating, cooling, and electromagnetic radiation exposure. In some embodiments, processing section 212 can include thermal cycling between one or more pre-determined temperatures for pre-determined durations as needed, for example, to perform PCR, or other amplification methods. In some embodiments, aqueous discrete volumes may continue to flow at a constant rate through processing section 212 while undergoing a desired process, or alternatively, they may dwell in a particular location in processing section 212. System 200, as illustrated in FIG. 2, includes another aqueous discrete volume characteristic determination and optional manipulation station 214. Aqueous discrete volumes 206 then flow through a junction J-1. In some embodiments, junction J-1 can be a T. As illustrated, fluid addition station 220 includes pump P-1 and valve V-1 in conjunction with a supply of different fluid (not shown) and can add that fluid to tube 204. In some embodiments, a gas phase can be introduced between aqueous discrete volumes 206. In some embodiments, an aqueous liquid can be added to aqueous discrete volumes 206 in junction J-1. In some embodiments, a different aqueous fluid can be added to a discrete volume between aqueous discrete volumes 206. An aqueous discrete volume characteristic determination and optional manipulation station 215, like 214 and 210 described above, follows liquid addition station 220. In some embodiments, station 215 evaluates the volume of liquid added to aqueous discrete volume 206.

Next in line, as illustrated in FIG. 2, is junction J-2. Junction J-2 and junction J-4, further down the line, fluidically connect back pressure unit 216 to pressurize tube 204 to a desired pressure. Between junctions J-2 and J-4, system 200 includes a second processing section 212, a junction J-3, at which point, fluid adding station 222 can add a volume of liquid to pre-existing aqueous discrete volumes 206, and an aqueous discrete volume characteristic determination and optional manipulation station 217 can evaluate the volume of liquid added to aqueous discrete volume 206.

As illustrated in FIG. 2, system 200 includes a final processing section 212, and processed aqueous discrete volumes are delivered from tube 204 to output station 218.

Reference will now be made to various embodiments of devices, apparatus, systems, and methods for controlling the fluid flow to manipulate immiscible-fluid, discrete volumes of a first fluid separated from one another by an immiscible spacing fluid, examples of which are illustrated in the accompanying drawings. Various embodiments of these can be used in the system described above with reference to Figs. 1And 1B. Wherever possible, the same reference numbers are used in the drawings and the description to refer to the same or like parts.

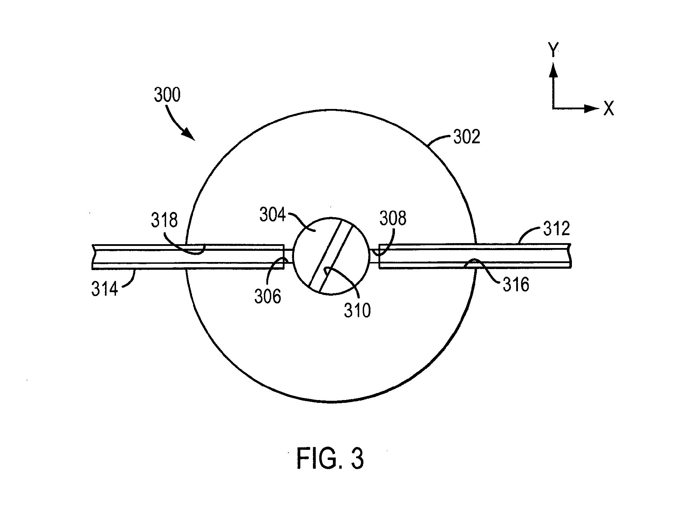

Referring now to Fig. 3, an embodiment 300 of a rotary valve that can work in any of the locations indicated in the system diagrams described above includes an annular stator 302 and a cylindrical rotor 304. Annular stator 302 has a first through-hole 306 and a second through hole 308, both of equal circular cross-sectional area, with their axes parallel, though they need not be, to the x-axis, and in alignment with each other. As illustrated, cylindrical rotor 304 has a through-hole 310 having a circular cross-sectional area the same shape and size of that both through-hole 306 or through-hole 308 and having its axis at an angle with the x-axis. As illustrated, valve 300 is in the closed position: through-hole 310 is not aligned with through-holes 306 and 308. In some embodiments, a larger cross-section counter bore 316 from the outer diameter of annular stator 302, and a larger cross-section counter bore 318 from the outer diameter of annular stator 302 are co-axial with through-hole 308 and through-hole 306, respectively, and tube 312, which can be, for example, PTFE capillary tubing, is disposed in counter bore 316 and tube 314, which can also be, for example, PTFE capillary tubing, is disposed in counter bore 318. In some embodiments, the inner cross-sectional shape and size of tube 312 matches the cross-sectional shape and size of through-hole 306. In some embodiments, the inner cross-sectional shape and size of tube 312 matches the cross-sectional shape and size of through-hole 310. In some embodiments, the inner cross-sectional shape and size of tube 314 matches the cross-sectional shape and size of through-hole 310. In this position, rotary valve 300 prevents the flow of fluid between tube 312 and tube 314 through through-hole 310. If cylindrical rotor 304 is rotated such that the axis of through-hole 310 is parallel with the x-axis, rotary valve 300 will permit the flow of fluid between tube 312 and tube 314 through through-hole 310. Any known mechanism providing rotary motion can be coupled to rotor 302 to actuate valve 300.

In some embodiments, annular stator 302 and cylindrical rotor 304 can be formed from PTFE. In some embodiments, annular stator 302 or cylindrical rotor 304 can be formed poly oxy-methylene, Nylon 66, Tefzel (a modified ETFE (ethylene-tetrafluoroethylene) fluoropolymer (manufactured by Du Pont)), Polytetrafluoroethylene (PTFE), Noryl classico family of modified PPE resins consists of amorphous blends of PPO* polyphenylene ether resin and polystyrene. (manufactured by General Electric), ultra high density polyethylene, polyphenol sulfide, and red Turcite TX or Turcite X ((Ethylene-chlorotrifluoroethlyene [ECTFE]) (manufactured by Busak+Shamban). In some embodiments, annular stator 302, cylindrical rotor 304, tube 312 and tube 314 comprise the same material. In some embodiments, at least one of annular stator 302, cylindrical rotor 304, tube 312, and tube 314 comprises a different material than at least the remaining listed components.

In some embodiments, conduits 312 and 314 are connected to annular stator 302 via fittings. In some embodiments, conduits 312 and 314 do not touch the cylindrical surface of cylindrical rotor 304, but fit in bores coaxially aligned with through-hole 306 or 308.

Use of rotary valve 300 does not displace a volume of fluid relative to its longitudinal location in tube 312 or 314. If a slug is located partially in one of conduits 312 and 314 and partially in through-hole 310, rotation of annular rotor 304 will split the slug into at least two parts. However, upon rotating annular rotor 304 back such that through-hole 310 aligns with conduits 312 and 314, the two or more volumes of the "split" slug will coalesce and the original slug can be flowed out of rotary valve 300.

Rotary valves as depicted in FIGS. 1A and 1B have multiple input tubes connected thereto. Rotary valve 300 described above can be modified to accomplish such a use. For example, multiple sets of aligned through-holes in annular stator 302 can accommodate additional tubes and the fluids contained therein, and which of the different fluids is passed through the rotary valve depends on the angular orientation of cylindrical rotor 304 relative to annular stator 302. As another possible modification, multiple through-holes could exist in rotor 304 at different angular orientations and different "Z" planes within rotor 304 than through-hole 310 illustrated in FIG. 3. A set of through-holes in stator 304 would align with each additional through-hole in rotor 304, and, accordingly, be at the appropriate "Z" value corresponding to the appropriate Z plane in rotor 304. The position of the holes in rotor 304 and stator 302 can be either aligned, with respect to the rotation of the rotor, with another set of holes in the rotor and stator such that both align at the same time, or they can align at different through-holes at a different angle. This permits the valves to be either opened and closed together, or separately.

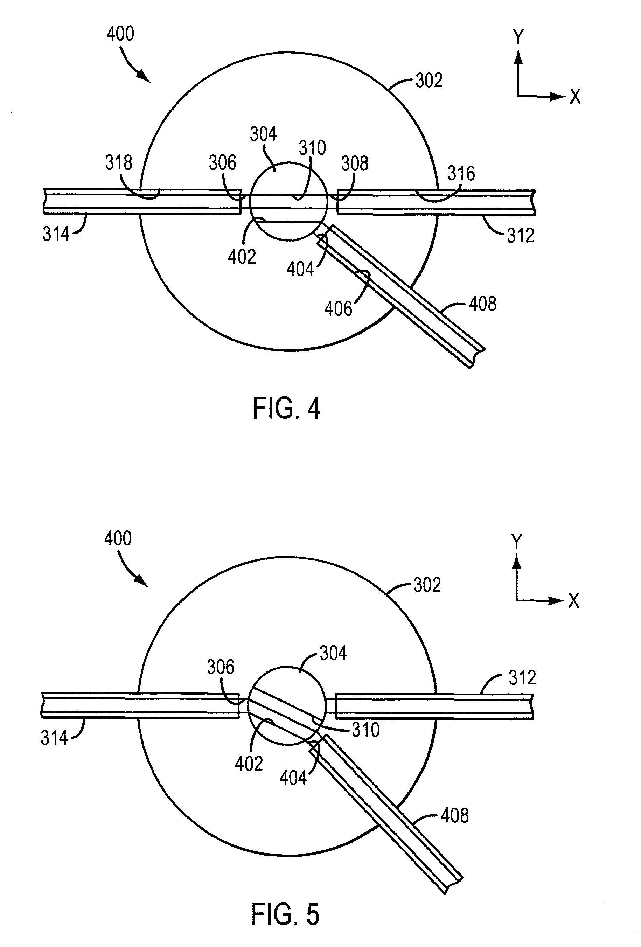

FIG. 4 illustrates a cross-sectional view in the XY plane of embodiment 400 of a rotary valve. As illustrated, and in some embodiments, rotary valve 400 can be a three-way valve. In other words, it can connect a first passageway with one of a second and third passageways. Rotary valve 400 is the same as rotary valve 300 except that cylindrical rotor 304 has a second through-hole 402 and annular stator 302 has a third through-hole 404 and third counter-bore 406 forming from the outer diameter of annular stator 302. A tube 408 is disposed in counter-bore 404. Second through-hole 402 is parallel to through-hole 310 and, as illustrated, when its axis is parallel to the x-axis, through-hole 402 is not aligned with any of the through-holes in annular stator 302. It is understood that second through-hole 402 does not have to be parallel, nor even entirely in the same plane as through-hole 310

FIG. 5 illustrates a cross-sectional view in the XY plane of the rotary value of FIG. 4, where the relative angular positions of cylindrical rotor 304 and annular stator 302 have changed such that through-hole 404 is in fluid communication with through-hole 314 and through-hole 404 of annular stator 302. In this position, rotary valve 400 permits flow of fluid between tube 314 and tube 408 through through-hole 402. As illustrated, and in some embodiments, through-hole 310 is not aligned, and therefore is not in fluid communication with either of tubes 312 or 314.

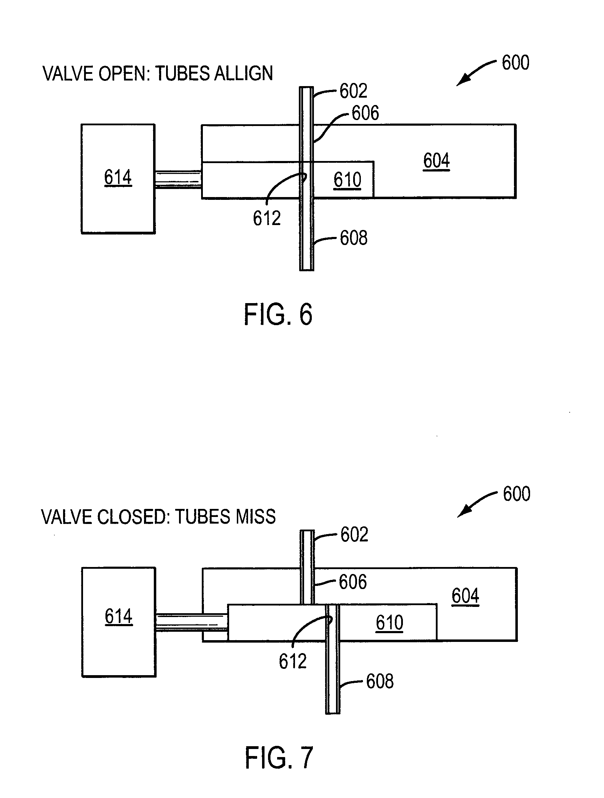

FIG. 6 illustrates a cross-sectional view in the XY plane of an embodiment 600 of a linear valve in an open position. In some embodiments, tube 602 is disposed in a through-hole 606 in member 604, which as illustrated in FIG. 6 is a block of rectangular shape. In some embodiments, a tube 608 is disposed in a through-hole 612 in member 610, which as illustrated in FIG. 6, can be a block of rectangular shape. As illustrated, tube 602 and tube 608 are axially aligned, and fluid can flow from one to the other. Member 610 is slidably mounted with respect to member 604. In some embodiments, a solenoid 614 can contact member 610.

FIG. 7 illustrates a cross-sectional view in the XY plane of the linear valve of FIG. 6 in a closed position. As illustrated, and in some embodiments, solenoid 614, or other motive force element, can apply a force to member 610 and slide it in the X direction with respect to member 604. Tube 608 and tube 602 no longer align, and, therefore, no fluid can flow between them, thus "closing" linear valve 600.

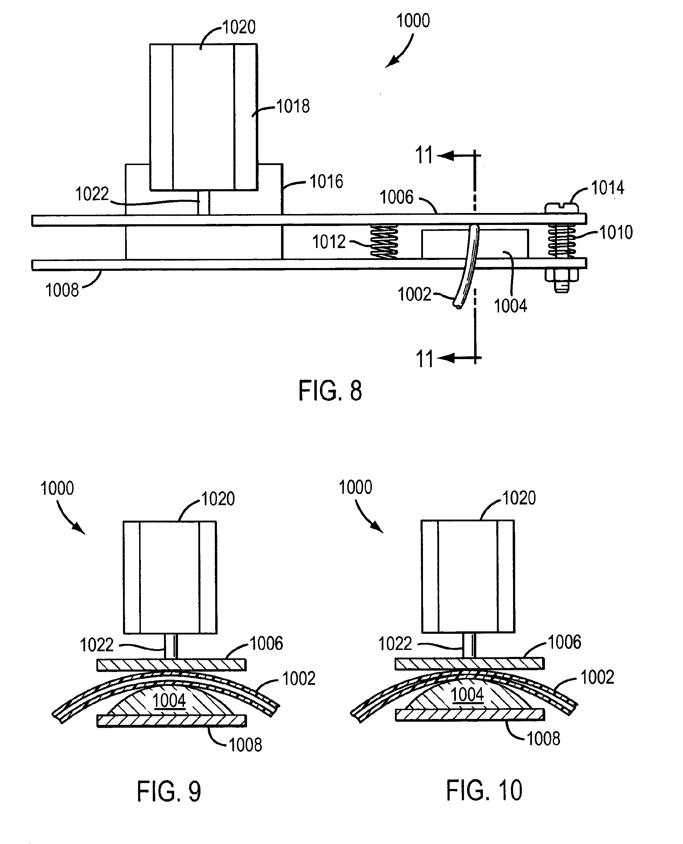

In some embodiments of a system such as that depicted in FIGS. 1A and 1B or FIG. 2, a pinch valve can be used to stop flow in conduits. FIG. 8 illustrates a side view of an embodiment 1000 of a pinch valve in an open position. Pinch valve 1000 provides a structure to collapse the inner cross-section of conduit 1002 until flow therein stops. In some embodiments, tube 1002 is between a radiused valve body 1004 and lever 1006. In some embodiments, radiused valve body 1004 is mounted to a base 1008. Springs 1010 and 1012 support lever 1006 a fixed distance above radiused valve body 1004. In some embodiments, spring 1010 is compressed between lever 1006 and base 1008 by a force applied to lever 1006 by fastener 1014. Fastener 1014, then, limits the distance between lever 1006 and base 1008. Lever 1006 is cantilevered beyond spring 1012. The minimum length that lever 1006 extends is determined by the required torque to rotate lever 1006 and the force supplied by the solenoid or other motive force element. In some embodiments, a solenoid can be mounted above lever 1006 to contact lever 1006 with its actuator in the un-actuated position.

FIG. 9 illustrates a front, cross-sectional view of the pinch valve of FIG. 8, more clearly illustrating the curvature of radiused valve body 1004 and the uncompressed tube 1002.

When solenoid 1020 actuates, actuator 1022 extends under force, rotating lever 1006 a pre-determined number of degrees, thereby collapsing or "pinching" tube 1002 between lever 1006 and radiused valve body 1004. FIG. 10 illustrates a front, cross-sectional view of the pinch valve of FIG. 8 in a closed position when tube 1002 is collapsed.

The ratio of volumes of the two smaller discrete volumes formed when a slug is split in a T-junction depends on the differences in the flow rate, diameter, and length of each of the two conduits flowing away from the T-junction. If the diameter and length are the same, then the ratio of the flow rates determines the ratio of the volumes of the two smaller, immiscible-fluid, discrete volumes.

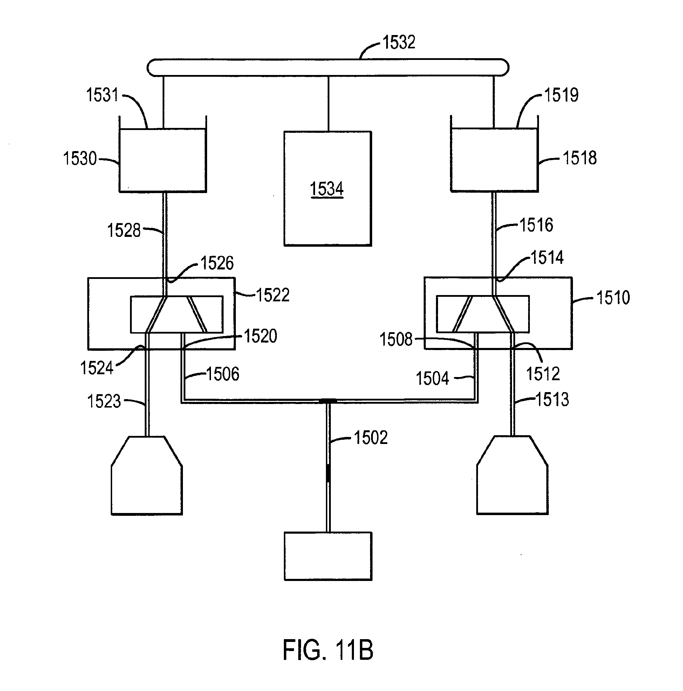

FIGS. 11A and 11B illustrate an embodiment 1500 of a system that can be used to split an immiscible-fluid slug into two, smaller, immiscible-fluid discrete volumes. In some embodiments, and as illustrated in FIGS. 11A and 11B, system 1500 includes a T-junction 1502, out of which fluid flows through tube 1504 in the positive X direction and through tube 1506 in the negative X direction. The other end of tube 1504 is connected to and is in fluid communication with port 1508 of a three- (or more) way valve 1510. Port 1512 of three-way valve 1510 is in fluid communication with tube 1513. Port 1514 of three-way valve 1510 is in fluid communication with tube 1516. As illustrated, three-way valve is positioned such that the internal pathway between port 1508 and port 1514 is open, and the internal pathway between port 1512 and port 1514 is closed. As illustrated, three-way valve 1510 is a linear three-way valve operated, for example, by a solenoid. Other three-way valve structures can be used. For example, the rotary three-way valve described in FIGS. 4-5 can be used. One end of tube 1516 is connected to and is in fluid communication with port 1514 of three-way valve 1510. The other end of tube 1516 is connected to and is in fluid communication with an outlet of a displacement pump 1518.

The other side of T-junction 1502 can be plumbed in a separate, but similar way. Thus, one end of tube 1506 connects to and is in fluid communication with T-junction 1502 and the other end is connected to and is in fluid communication with port 1520 of three-way valve 1522. One end of tube 1523 is connected to and is in fluid communication with port 1524 of three-way valve 1522. Port 1526 of three-way valve 1522 is connected to and is in fluid communication with one end of tube 1528. The other end of tube 1528 is connected to and is in fluid communication with an outlet of displacement pump 1530.

Displacement mechanism 1531 and 1519 can be moved at the desired speed, in the same direction, and starting at the same time to create a desired ratio of flow rates through the fluid pathway between an outlet of T-junction 1502. If the movement does not start at the same time, in the same direction, or at the desired speed, the ratio of flow rates in each divergent pathway may not be as desired, resulting in an undesired volumetric split of a slug in T-junction 1502.

To reduce the likelihood of an undesired volumetric split, the displacement mechanisms of displacement pumps 1530 and 1518 can both be moved by a single motion. As illustrated in FIGS. 11A and 11B, one structure for moving displacement mechanism 1531 at the same time, and at the same speed is a link 1532 rigidly coupled to each of displacement mechanisms 1531 and 1519 and to the linear output of a motor 1534. In some embodiments, the ratio of flow rates can be changed by changing the ratio of the cross-sectional areas of displacement pumps 1530 and 1514. Thus, for even splitting of slugs, in some embodiments, the cross-sectional area of displacement pump 1530 equals the displacement area of displacement pump 1518. In some embodiments, to prevent bubbles, the movement of pumps 1530 and 1518 can permit flow from additional positive pressure pump feeding into tee 1502 from tube at "Fig. 11A" (not shown); thus negative pressures are never created.

After motor 1534 has moved link 1532 the desired distance in the positive Y direction, it stops. Three-way valves 1510 and 1522 change position to close internal pathways between ports 1526 and 1520 and 1514 and 1508, respectively, and open internal pathways between ports 1526 and 1524 and 1514 and 1512, respectively. FIG. 11B illustrates three-way valves 1522 and 1510 in this position. Motor 1534 moves link 1532 in the negative Y direction and fluid in conduits 1528 and 1516 flow through three-way valve 1522 and 1510, respectively, into conduits 1523 and 1513, respectively. In some embodiments, tube 1523 and tube 1513 can be in fluid communication with a waste reservoir.

The configuration of displacement pumps, three-way valves and connected conduits described above can also be used to control the volumes of miscible fluid combined in a T, if the flow directions are reversed, along with the steps. In other words, the three-way valves start in the position shown in FIG. 11B and the motor then moves in the positive Y direction, pulling a volume of fluid through conduits 1523 and 1513, respectively, through the respective three-way valve and into tube 1528 and 1516, respectively. The three-way valve then changes position from that illustrated in FIG. 11B to that illustrated in FIG. 11A, and the motor then moves in the negative Y direction, pushing a desired volume of fluid through each of conduits 1528 and 1516, through the respective three-way valve and into tube 1506 and 1504, respectively. The two streams of fluid then combine in preset volumetric ratio and exit T-junction 1502. One use for such a configuration and method of operation is for combining two liquid reagents prior to forming discrete volumes to coalesce with existing discrete volumes. Referring to FIG. 1B, such a configuration and method of operation could be used in at least at cross-junction 86 and cross-junction 88 to add dye terminators and forward or reverse primers to existing immiscible-fluid, discrete volumes. In some embodiments, the intake to the pump can be between 90 and V10 and 92 and V11.

The method of operation of the configuration described immediately above can also generate immiscible-fluid discrete volumes of a first fluid in a consistent ratio with a second fluid with which it is immiscible. Thus, referring to FIGS. 1A and 2, such a configuration could be used in at least at T-junction 66 and in supply unit 202.

Another method of use can combine slugs as in the zipper approach to merging samples and primer sets. After creation of slugs sets of primers in tubes 1523 and 1513 they can be pulled into 1528 and 1516, and then pushed so that they merge in tube 1502. A pair of detectors can monitor the position of the slugs, oil adding devices can be used to add oil into tubes 15061504 so that the slugs will meet precisely.

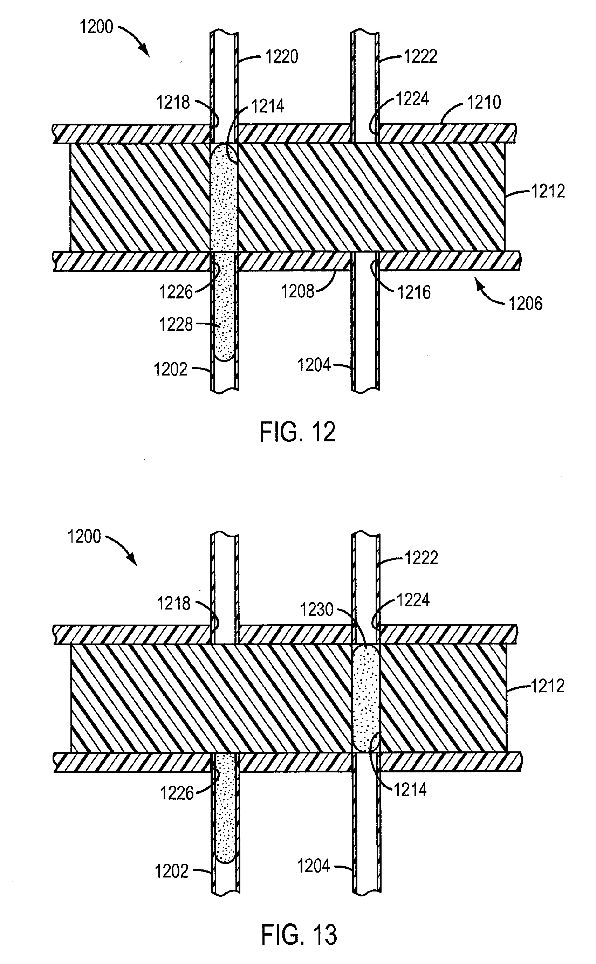

Another system for splitting aqueous immiscible-fluid, discrete volumes spaced apart by spacing fluid, in a conduit, is depicted in FIGS. 12 - 16. As shown, the system 1200 comprises an immiscible-fluid-discrete-volume supply tube 1202 and a spacing fluid supply tube 1204 which deliver immiscible-fluid, discrete volumes in spacing fluid and spacing fluid, respectively, to a housing 1206. System 1200 can be used to form immiscible-fluid, discrete volumes of any first fluid separated from one another by an immiscible second fluid. Each of supply lines 1202 and 1204 can be operatively connected through a respective delivery unit (not shown) that can comprise, for example, a pump and a fluid reservoir. Housing 1206 houses a slider 1212 therein, which is configured for sliding movement in housing 1206. Slider 1212 is provided with a through hole 1214 that, in the position shown in Fig. 12, is aligned with immiscible-fluid-discrete-volume supply tube 1202. Any suitable drive unit can be provided for effecting sliding movement of slider 1212 in housing 1206, for example, a programmable drive unit. Slider 1212 is snugly seated in housing 1206 and causes a sealing action to seal off spacing supply tube 1204, when positioned as shown in Fig. 12A.

Housing 1206 is provided with an upper wall 1210 and a lower wall 1208. Lower wall 1208 is provided with through holes 1226 and 1216 to accommodate and/or provide a fluid communication with aqueous immiscible-fluid-discrete-volume supply tube 1202 and spacing-fluid supply tube 1204, respectively. Upper wall 1210 is provided with through holes 1224 and 1218 to accommodate and provide fluid communication with a right-split-slug tube 1222 and a left-split-slug tube 1220, respectively. In the position shown in Fig. 12, a slug 1226 has been flowed upwardly through immiscible-fluid-discrete-volume supply tube 1202 to fill through-hole 1214 of slider 1212 with half of its volume. The forward most point of slug 1226 is lower than upper wall 1210. For even splitting of slug 1226, as illustrated, the midpoint of slug 1226 is positioned at the interface of lower wall 1208 and slider 1212. In some embodiments, slug 1226 can be held in position by an electric field, regardless of whether oil flows past it.

In the next step of a method using system 1200, slider 1212 is shifted to the right-side position shown in Fig. 13. As shown in Fig. 13, in the right-side position, slider 1212 seals off immiscible-fluid-discrete-volume supply tube 1202 and left-split-slug tube 1220. Furthermore, when in the right-side position shown, through-hole 1214 of slider 1212 lines up with spacing fluid tube 1204 and right-split-slug tube 1222, as well as with respective through-holes 1224 and 1216. As illustrated, because spacing-fluid supply tube 1204 contains spacing fluid, the volume of liquid that used to be half of slug 1226 forms as a new right split slug 1230.

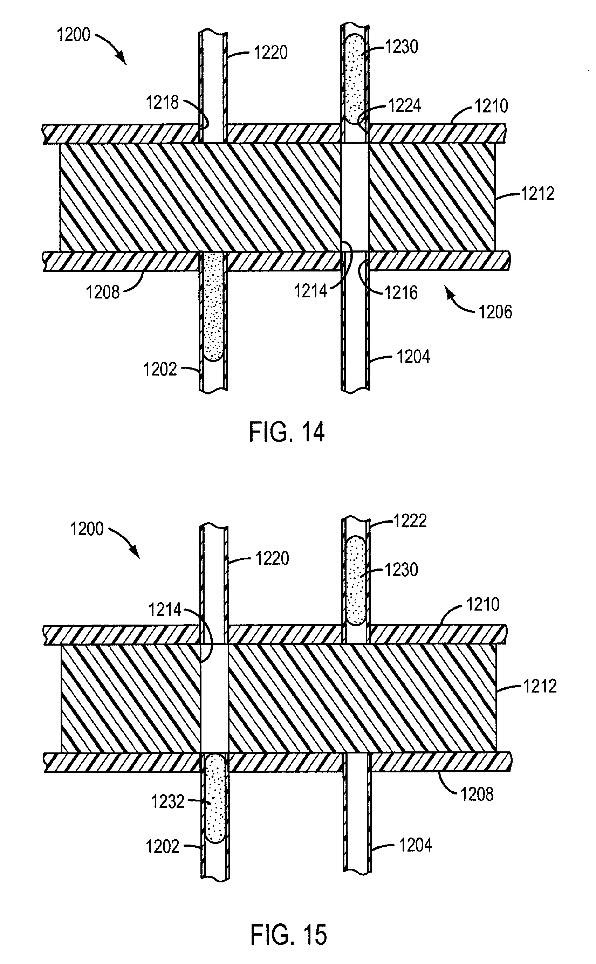

Next, spacing fluid is flowed upwardly through the spacing-fluid supply tube 1204 to move a slug 1230 out of through-hole 1214 and into right-split-slug tube 1222, as shown in Fig. 14. In some embodiments, enough spacing: fluid is flowed through through-hole 1214 to completely flow slug 1230 from through-hole 1214 and past upper wall 1210 of housing 1206. As illustrated, through-hole 1214 is filled with spacing fluid in FIG. 14.

Subsequently, slider 1212 is moved back to the left-side position as shown in Fig. 15 so that through-hole 1214 of slider 1212 can then be again aligned with immiscible-fluid-discrete-volume supply tube 1202 and left-split-slug tube 1220. Because through-hole 1214 is filled with spacing fluid, the volume of fluid that used to be the remaining half of slug 1226 forms a smaller slug 1232.

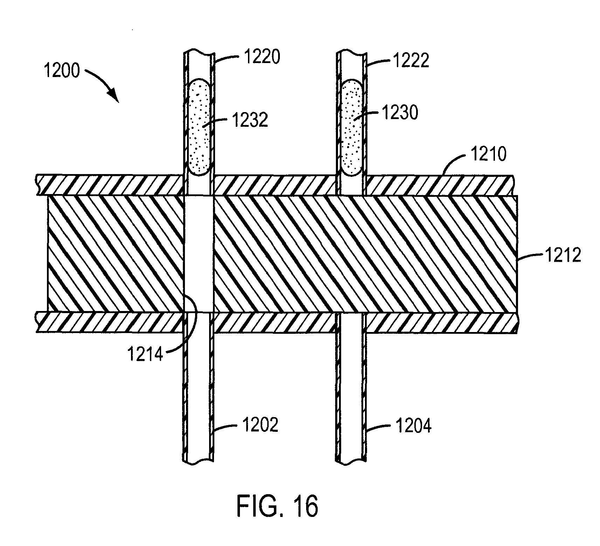

Thereafter, as shown in Fig. 16, fluid in immiscible-fluid-discrete-volume supply tube 1202 is flowed upwardly to completely flow slug 1232 through through-hole 1214 and into left-split-slug tube 1220 and, in some embodiments, above upper wall 1210. In some embodiments, through-hole 1214 becomes filled with spacing fluid, depending on the spacing between slugs in immiscible-fluid-discrete-volume supply tube 1202. As illustrated, through-hole 1214 is filled with spacing fluid.

Thereafter the series of steps described in conjunction with FIGS. 12-16 can be repeated. The ratio of post-split slug volumes 1230 and 1232 is determined by the positioning of slug 1226 with respect to the interface between lower wall 1208 and slider 1212 and the height of slider 1212.

In some embodiments, the initial immiscible-fluid-discrete-volume can be flowed into through hole 1214 such that part of it remains in through-hole 1214, and part of it extends into left-split-part conduit 1220. Then, when slider 1212 is moved back to align with conduit 1220 after "splitting" slug 1228, slug 1232 can be moved further into conduit 1220 with less, if any, spacing fluid flowing through through-hole 1214.

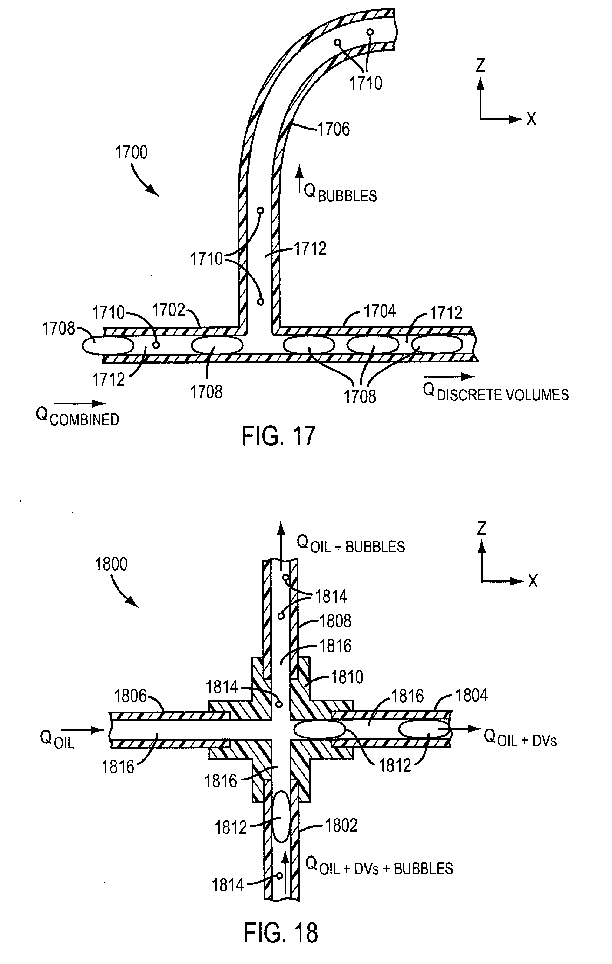

FIG. 17 illustrates an embodiment 1700 of a bubble remover, which takes advantage of the buoyancy of gas in a spacing fluid to remove undesired bubbles from spacing fluid between immiscible-fluid, discrete volumes. As illustrated in FIG. 17, conduits 1702, 1704, and 1706 form a junction in the shape of T. As illustrated, tube 1702 contains two slugs 1708 and a gas-phase discrete volume (herein after referred to as a bubble) 1710 in a spacing fluid 1712 flowing at a rate, Q combined. As illustrated, tube 1704 contains two whole slugs and a part of a third slug 1708 in spacing fluid 1712 flowing at a rate, Q discrete volumes. Note that spacing fluid 1712 in tube 1704 is free of bubbles 1710. As illustrated, tube 1706 contains two bubbles 1710 in spacing fluid 1712 flowing at a rate, Q bubbles. Bubbles 1710 in tube 1706 may have a speed greater than the speed of spacing fluid 1712 due to the differences in specific gravity (buoyancy) until they reach the top of the bend in tube 1706. As the diameter of each tube 1702, 1704, and 1706 is the same in FIG. 17, slugs 1702 flow into tube 1704 after the junction due to the difference in flow rates between Q discrete volumes and Q bubbles. In some embodiments, the three flow rates can have the following relationship: Q combined > Q discrete volumes > Q bubbles. In some embodiments, flow rate Q combined can be 10 times as great as Q bubbles and can be up to 100 times as great As illustrated, slugs 1708 in tube 1704 are separate by less distance than in tube 1708 due to the movement of spacing fluid into tube 1706.

In some embodiments, conduit 1702 and 1704 can form an angle of about 90 degrees and conduit 1702 and conduit 1706 can form an angle of about 180 degrees. In this embodiment, slugs bend approximately ninety degrees as the move from the junction of conduits 1702 and 1704. Gas bubbles 1710 flow straight up from conduit 1702 into conduit 1706.

FIG. 18 illustrates another embodiment 1800 of a bubble remover. Bubble remover 1800 includes a "cross" junction connected to conduits 1802, 1804, 1806, and 1808. As illustrated, tube 1802 contains an immiscible-fluid, discrete volume, depicted as a slug 1812, and a gas-phase discrete volume, depicted as a bubble 1814 in spacing fluid 1816 flowing at a rate, Q oil + DVs + bubbles, in the positive Z direction. As illustrated, tube 1804 contains two slugs 1812 in spacing fluid 1816 flowing at a rate, Q oil + DVs, in the positive X direction. Tube 1804 is free of bubbles 1814. As illustrated, tube 1806 contains spacing fluid 1816 flowing at a rate, Q oil, in the positive X direction. As illustrated, tube 1808 contains three bubbles 1814 in spacing fluid 1816 flowing at rate, Q oil + bubbles, flowing in the positive Z direction. As a bubble 1814 reaches the junction volume, it transfers from the stream of spacing fluid 1816 from tube 1802 to the stream of spacing fluid 1816 in tube 1808 due to buoyancy. In some embodiments, Q oil + DVs + bubbles is equal to Q oil + DVs. In some embodiments, Q oil + DVs+ bubbles is greater than Q oil + DVs.

Application of additional pressure to the system can cause the gas phase to be dissolved into solution, which is in effect, another embodiment of a bubble remover. Such pressure is sometimes called "back pressure." The use of back pressure can be useful during batch thermal cycling of the fluids can apply to all thermal cycling processing sections, whether in thermal spirals, for example, in a system as illustrated in FIG. 1, or in a flow through system, to prevent movement of discrete volumes that may coalesce if in contact. If no back pressure is applied, pre-existing bubbles between two slugs may expand and cause one or more of those slugs to contact, and, therefore, merge with, another slug, reducing the desired separation of reactions within each slug. During thermal cycling, without back pressure, bubbles may form in previously bubble-free spacing fluid, which may then expand and cause merging of slugs.

FIG. 19 illustrates an immiscible-fluid-discrete-volume processing apparatus 1900 including an embodiment of a back-pressure device. Apparatus 1900 includes a tube 1902 containing two or more discrete volumes of aqueous liquid 1904, each of which is in contact with an immiscible, non-aqueous liquid 1906. As illustrated, tube 1902 is blocked at one end by a valve in a closed position or other pressure retaining structure, illustrated here as a box 1908. Tube 1902 is connected to, as illustrated, and in fluid communication with, a reservoir 1910 containing a volume of the immiscible, non-aqueous liquid 1906. In some embodiments, reservoir 1910 can be expandable and contractible. In some embodiments, and as illustrated here, the immiscible, non-aqueous liquid 1906 is in contact with a diaphragm 1912, which is between reservoir 1910 and an enclosure 1914. In some embodiments, enclosure 1914 is filled with pressurized fluid 1916. In some embodiments, pressurized fluid 1916 is an inert gas. In some embodiments, pressurized fluid 1916 is air.

In some embodiments, the pressure of pressurized fluid 1916 is supplied by a pressure pump 1922. In some embodiments, the pressure of pressurized fluid 1916 is regulated to a set pressure by regulator 1920 connected between pressure pump 1922 and enclosure 1914. In some embodiments, and as illustrated in FIG. 19, a three-way valve 1918 may be connected between enclosure 1914 and regulator 1920 or pressure pump 1922. In some embodiments, and as illustrated, the common port of three way valve 1918 is connected to reservoir 1914 and is in fluid communication with the space between enclosure 1914 and diaphragm 1912. The first of the two input/output ports can be in fluid communication with regulator 1920, if present in the apparatus, or with pressurized pump 1922. The second of the two input/output ports can be in fluid communication with the external atmosphere, for releasing the pressure in the space between enclosure 1914 and diaphragm when back pressure is not intended.

FIG. 20 illustrates another embodiment of an immiscible-fluid-discrete-volume processing apparatus 2000 including an embodiment of a back-pressure device. Apparatus 2000 includes a tube 1902 containing two or more discrete volumes of aqueous liquid 1904, each of which is in contact with an immiscible, non-aqueous liquid 1906. As illustrated, tube 1902 is blocked at least one end by a valve in a closed position or other pressure retaining structure, illustrated here as a box 1908, or box 2002. As illustrated, tube 1902 is positively pressurized by being in fluid communication with a back pressure device as illustrated in FIG. 19 (for convenience, not all structures discussed in conjunction with FIG. 19 are illustrated in FIG. 20) at least at a first location in tube 1902. One location on tube 1902 in fluid communication with reservoir 1910 is at the junction 2004 with tube 2006. A second location on tube 1902 in fluid communication with reservoir 1920 is at junction 2008 with tube 2010. Between junctions 2004 and 2008, tube 1902 is heated or cooled or maintained at one or more temperatures by temperature-control unit 2012. Temperature-control unit 2012 can comprise, for example, a heating unit, a cooling unit, an isothermal unit, a thermal-cycler. While illustrated as contacting temperature-control unit 2012 in a single straight-line segment, tube 1902 may form, for example, a coil around the external perimeter of temperature-control unit 2012, a spiral of decreasing radius on one surface, or a serpentine pattern across one or more surfaces of temperature-control unit 2012.

FIG. 21 illustrates a discrete volume characterization determination and optional manipulation control system 2100. System 2100, as illustrated, includes sources 2102 and 2104. Sources 2102 and 2104 can be optical sources, such as a laser, an LED, a high intensity white light source, such as for example, a tungsten or a halogen lamp, with or without a band pass filter, positioned near conduit 2106. In some embodiments, sources 2102 and 2104 can be a single source, configured to illuminate two or more different areas. Conduit 2106 contains a spacing fluid 2108 which is immiscible with the fluid in immiscible-fluid, discrete volumes 2114 and 2112, which are depicted as slugs. System 2100, as illustrated, includes detectors 2116 and 2118, which receive signals from sources 2102 and 2104, respectively, as modified by slugs 2114 and 2112, respectively. Detectors 2116 and 2118 can be, for example, fluorescence detectors or infra red detectors. In some embodiments, detectors 2116 and 2118 can each be a photo multiplier tube (PMT) or an infra red, refraction index detector. In some embodiments, only one source and detector are needed to gather desired characteristics of aqueous discrete volumes.

Detectors 2116 and 2118 are coupled to sense circuitry 2120 and 2122, respectively, which senses the detected signal and provides it in a useable format to a controller 2124. Controller 2124 can include for example, a set of instructions, memory, and a processing unit. Controller 2124 can communicate with one or more valves 2126, which permit or prevent flow upstream or downstream of a detector, one or more pumps 2128 and 2130, which pressurize (negatively or positively), displace, or otherwise move fluid upstream and/or downstream of a detector, motors 2132 to move the relative locations of, for example, containers of liquids, and conduits for removing liquid from or adding liquid to those containers, and one or more monitors for viewing and further interface with the presented information. In communicating, controller may direct a component to maintain or change its state based on the signal received from sense circuitry 2120 and/or 2122.

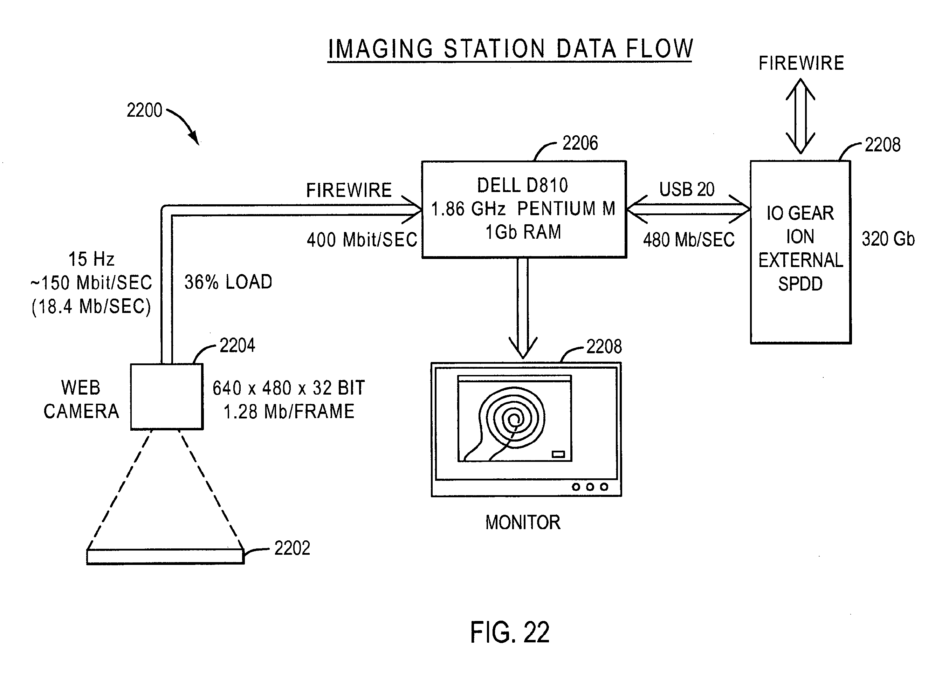

FIG. 22 illustrates an imaging station data flow of system 2200. In some embodiments, a detector can comprise a camera to gather characteristics of aqueous discrete volumes in an immiscible spacing fluid within a conduit. As illustrated, conduit 2202 contains aqueous discrete volumes of interest (not shown), which is imaged by camera 2204. Camera 2204 can transmit the image data to a computer 2206 via Firewire. Computer 2206 transmits the image recorded by camera 2204 as optionally modified by software, to monitor 2208. Computer 2206 can also transmit data to an external memory device, such as, for example, a hard disk drive. As illustrated in the image displayed on monitor 2208, conduit 2202 is shaped in a spiral. In some embodiments, this image data can be use in monitoring PCR to provide real-time quantization of expression within each discrete volume of aqueous sample. In some embodiments, this image data can be used to estimate what percentage of discrete volumes contained DNA before and/or during to amplification.

In some embodiments, a method is provided that can comprise using the system described herein to process an aqueous immiscible-fluid-discrete-volume. Additional compounds may be needed in the aqueous discrete volume during the processing and, as discussed above, the spacing of adjacent aqueous discrete volumes may need to change to accommodate the addition of liquids comprising the desired compounds involved in the reaction or processing within an aqueous discrete volume.

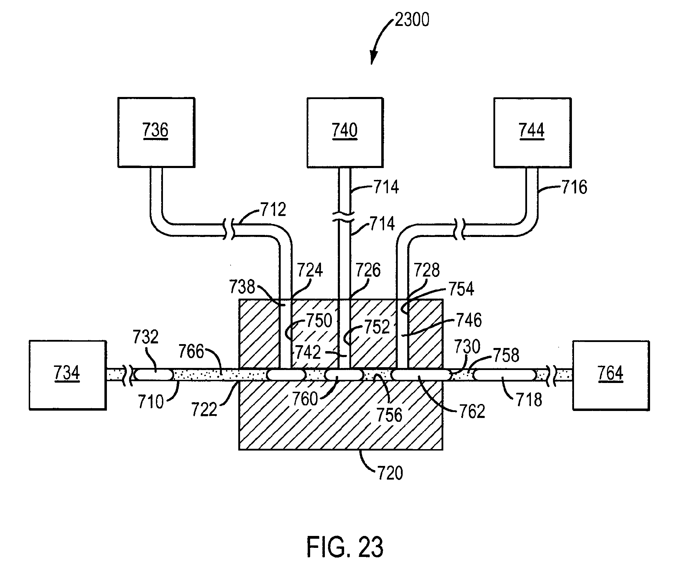

According to various embodiments of the present teachings illustrated, for example, in Fig. 23, a plurality of tubes 710, 712, 714, 716, and 718, can be connected to, or otherwise be in fluid communication with, a manifold 720 at a plurality of respective openings 722, 724, 726, 728, and 730. While tubes 710, 712, 714, 716, and 718 are shown terminating at manifold 720 and in fluid communication with respective passageways in manifold 720, it is to be understood that the tubes can, in some embodiments, extend into manifold 720. For example, instead of the arrangement shown in Fig. 23, tubes 712, 714, and 716 can be inserted into bores formed in manifold 720 and which extend all the way to respective junctions with immiscible-fluid-discrete-volume channel 756, or closely adjacent to immiscible-fluid-discrete-volume channel 756. Manifold 720 is shown in cross-section and tubes 710, 712, 714, 716, and 718 are depicted as transparent, although they do not have to be, so that the oil and reagents therein can be seen in the figure. Tube 718 can be transparent in some embodiments such that a reaction involving an aqueous immiscible-fluid-discrete-volume, or the position of an immiscible-fluid-discrete-volume can be monitored inside tube 718 can be detected from outside tube 718. In the embodiment shown in Fig. 7, tube 710 is shown as a supply tube that supplies pre-formed immiscible-fluid, discrete volume 732 in spacing fluid 766 to manifold 720. Immiscible-fluid, discrete volume 732 can be moved from a supply unit 734. In some embodiments, supply unit 734 can include a temporary storage tube of pre-formed discrete volumes of a first type of fluid in contact with a second type of fluid with which it is immiscible. In some embodiments, supply unit 734 can include an immiscible-fluid-discrete-volume-forming apparatus. Supply unit 734 can also include a discrete-volume characterization determination and optional manipulation apparatus (not shown) as described in conjunction with FIGS. 21 and 22.

According to the various embodiments, manifold 720 can comprise a fluorocarbon material, for example, a perfluorocarbon material such as polytetrafluoroethylene. In some embodiments, manifold 720 can comprise the same material as is used for the tubes 710, 712, 714, 716, and/or 718, or other materials known to those skilled in the art. Materials can be selected that are non-reactive or minimally reactive with the liquids passing through manifold 720.

According to various embodiments, tube 710 can be connected to manifold 720 by any appropriate connection, for example, using a fitting or connector that extends from manifold 720, by frictionally fitting tube 710 into a bore formed in manifold 720 wherein the bore has an inner diameter that is about equal to the outer diameter of tube 710, or by using an adhesive, or the like. Similarly, tubes 712, 714, 716, and 718, can be connected to manifold 720.

In the embodiment shown, tube 712 is connected at a first end to manifold 720 and at an opposite, second end to a first liquid supply unit 736. First liquid supply unit 736 can comprise, for example, a supply of a first liquid 738 and a pump for moving first liquid 738 into and through tube 712. First liquid supply unit 736 can comprise a pump of the same type, or of a different type, as the type used for supply unit 734. Controller 2124 (shown in FIG. 21) can control the flow rate of first liquid 738, whether a result of differential pressure or displacement or other motive force. In some embodiments, controller 2124 can control the pressure independently of the other supply units in the system.

A tube 714 can be connected at a first end to manifold 720 and at a second, opposite end, to a second liquid supply unit 740. Second liquid supply unit 740 can comprise, for example, a supply of a second liquid and a pump for moving the second liquid 742 into and through tube 714. Second liquid supply unit 740 can comprise a pump that can be the same type as, or different than, the type of pump used in supply unit 734. Controller 2124 (shown in FIG. 21) can control the flow rate of second liquid 742, whether a result of differential pressure or displacement.

A tube 716 can be connected at a first end to manifold 720 and at a second, opposite end to a third liquid supply unit 744. Third liquid supply unit 744 can comprise, for example, a supply of a third liquid 746 and a pump for moving third liquid 746 into and through tube 716. The pump can be the same type as, or different than, the type of pump used for supply unit 734. Controller 2124 can control the flow rate of third liquid 746 in tube 716, whether a result of differential pressure or displacement/time or other motive force..

According to various embodiments, first liquid 738, second liquid 742, and third liquid 746, can each comprise an aqueous medium, for example, an aqueous solution, and each can be miscible with the other two reagents. In some embodiments, each of first liquid 738, second liquid 742, and third liquid 746, can be immiscible with spacing fluid 766 in contact with aqueous discrete volume 732. In some embodiments, the first liquid 738, second liquid 742, or third liquid 746 can comprise spacing fluid 766 to increase or decrease the spacing between immiscible-fluid discrete volumes prior to receiving an additional volume of aqueous liquid at a subsequent junction. Controller 2124 (see FIG. 21) can control the speed and direction of pumps in liquid supply units 736, 740, and 744 according to a set program and as optionally modified with data from detectors upstream and/or down stream of additional liquid addition and/or removal. As such, as the first liquid 738, second liquid 742, and third liquid 746 pass through passageways 750, 752, and 754, respectively, and flow into immiscible-fluid-discrete-volume channel 756 in the body of manifold 720.

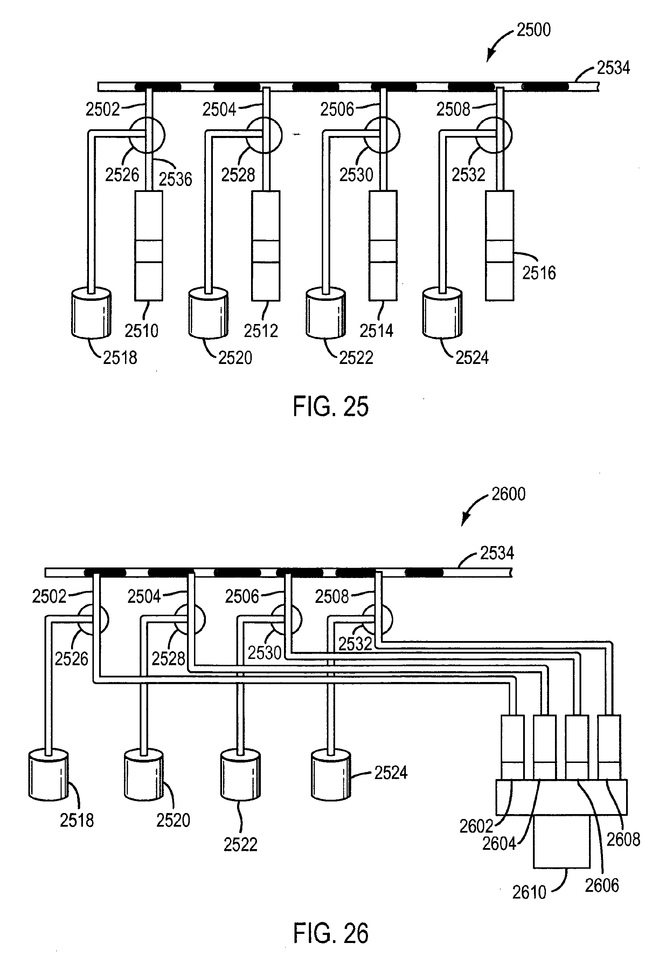

In an exemplary embodiment, a first aqueous immiscible-fluid-discrete-volume 732 flowing through immiscible-fluid-discrete-volume channel 756 can be supplemented with second liquid 742 flowed out at a constant rate or injected by second liquid supply unit 740 as aqueous immiscible-fluid-discrete-volume 732 lines-up with the junction of passageway 752 and immiscible-fluid-discrete-volume-forming channel 756. As a result, a supplemented immiscible-fluid-discrete-volume 760 can be formed that comprises a mixture of the aqueous liquid of aqueous immiscible-fluid, discrete volume 732 and second liquid 742. As supplemented immiscible-fluid-discrete-volume 760 proceeds through immiscible-fluid-discrete-volume-forming channel 756 and reaches the junction of immiscible-fluid-discrete-volume-forming channel 756 with passageway 754, third liquid 746 can be flowed out at a constant rate or injected from third liquid supply unit 744, when properly timed, to form a further supplemented immiscible-fluid-discrete-volume 762 comprising a mixture of the aqueous liquid of aqueous immiscible-fluid, discrete volume 732, second liquid 742, and third liquid 746. It is to be understood that additional tubes can be connected to additional respective passageways (not shown) if it is desired to provide such additional features in a system. It is to be understood that system 2300 can also comprise a separate manifold for each junction of a liquid adding or removal conduit and an immiscible-fluid-discrete-volume supply conduit, adjacent manifold being connected by tubes. While the manifold is not illustrated, such a concept as just mentioned is illustrated in FIG. 25, which illustrates junctions of four liquid-adding and/or liquid removal conduits with an immiscible-fluid-discrete-volume supply conduit.

Such an embodiment that permits the addition of one or more different miscible liquids to be added to an immiscible-fluid-discrete-volume, can assist in adding a first individual primer to a selected slug at a first junction, and a second individual primer to a selected slug at a second junction. After receiving both primers, the selected slug will contain a desired primer set for amplification in a later section. In this way, the number of different primer liquids needed to provide a predetermined number of primer pairs for requencing methods, such as those described in conjunction with SYSTEM APPLICATION, can be reduced. Table 3 illustrates the combination of eight different primer liquids to provide 16 different primer pairs for use with a set of slugs, or other discrete volumes.

**Table 3**

| M x N Zip code Primers | | | | | |
|---|---|---|---|---|---|
| 3' primer | | | | | |
| | | A | B | C | D |
| | A | AA | AB | AC | AD |
| | B | BA | BB | BC | BD |
| 5' primer | C | CA | CB | CC | CD |
| | D | DA | DB | DC | DD |

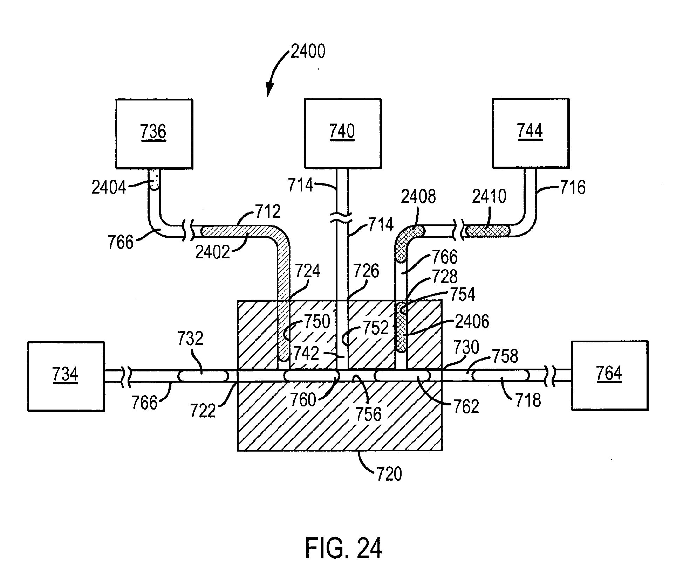

Referring again to FIGS. 23 and 24, an outlet from manifold 720 can be provided at opening 730 and can be connected to tube 718, for example, using a connection as described above. In an exemplary embodiment, tube 718 is connected by a first end to manifold 720, and at a second, opposite end, to a collection unit 764 where the further supplemented immiscible-fluid-discrete-volumes 762 can be processed, detected, or otherwise manipulated, analyzed, and/or transferred to another device or system.

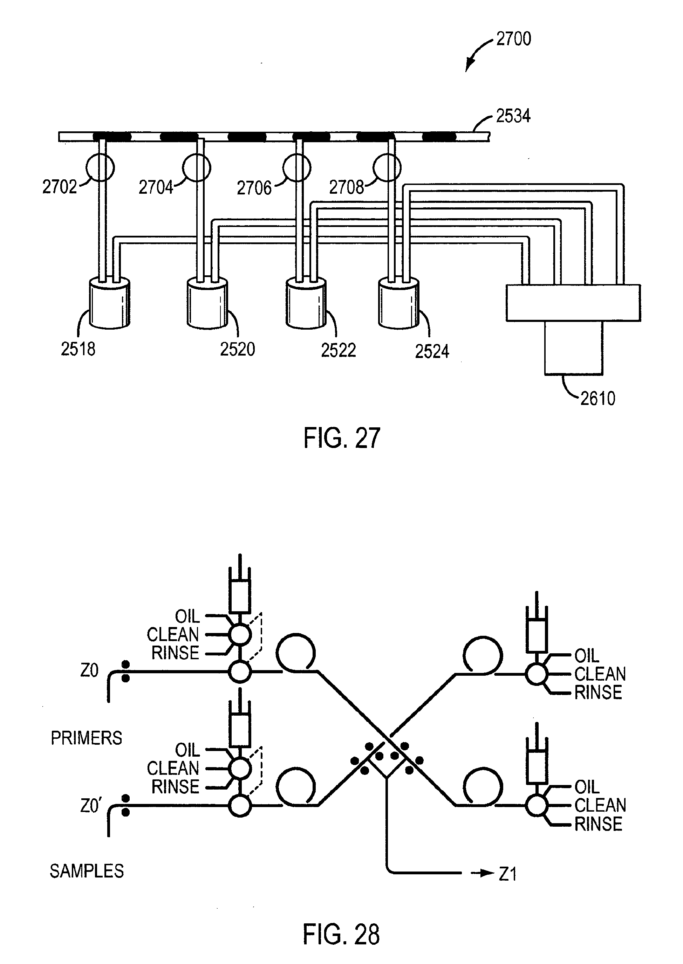

According to various embodiments, system 2300 shown in Fig. 23 can comprise a single pump and a valving scheme that replaces the four individual pumps described above in connection with units 734, 736, 740, and 744. Embodiments of two such single pump and valving schemes are illustrated in FIGS. 26 and 27 and will be described below in conjunction with those figures

System 2400 illustrated in FIG. 24 includes the same solid structures as FIG. 23, but the liquids in tubes 712 and 716 are configured differently. For example, tube 712 contains two distinct types of aqueous liquids, each in a discrete volume, namely, first liquid 2402 and second liquid 2404, spaced apart by spacing fluid 766. The volume of first liquid is sufficient, as illustrated, to add a pre-determined amount to several immiscible-fluid, discrete volumes flowing past the junction with immiscible-fluid-discrete-volume supply channel 756. When all of first liquid 2402 has been flowed out of 736, spacing fluid 766 will advance into immiscible-fluid-discrete-volume supply channel 756, increasing the amount of spacing fluid between discrete volumes. Then, if the flow through tube 712 continues, second liquid 2404 can be added to immiscible-fluid, discrete volumes flowing past the junction of passageway 750 and channel 756. If the discrete volumes are slugs, then tube 712 and passageway 750 are not contacted by the first or second liquids, reducing the chance that second liquid 2404 will be contaminated by first liquid 2402.

As illustrated, tube 716 and passageway 754 contain a third liquid 746 (which number is not in the figure) in three separate discrete volumes 2406, 2408, and 2410 separated by spacing fluid 766. As illustrated, the volumes of third liquid 746 in volumes 2406, 2408, and 2410 is roughly equal to the volume of the passing immiscible-fluid, discrete volumes 732 in through-hole 756. By controlling the flow rates in each conduit, discrete volumes, 2406, 2408, and 2410 can each coalesce with a discrete volume 732 to form a supplemented (not illustrated) discrete volume of combined liquids 732 and 746. In some embodiments, a particular liquid is desired to be added to only certain slugs, and having preformed volumes of the addition liquid can accomplish this task with minimal contamination of the slugs to which no addition liquid is to be added with that addition liquid, as may be the case when the addition liquid is present up to the junction of the addition conduit and the main conduit, even if its flow rate is zero.

While the methods described in conjunction with Fig. 23 involve adding more than one of the first, second, and third reagents, it is to be understood that separate control units, or a single controller 2124 (See FIG. 21), described herein can be used to control the addition of just one, two, or all three, of those reagents. For example, if it is desired to add only the second liquid to an aqueous immiscible-fluid-discrete-volume, the control units for supply units 736 and 744, or controller 2124 (see FIG. 21) can control those units not to add the first and third reagents to that aqueous immiscible-fluid-discrete-volume.

According to various embodiments, different approaches or mechanisms can be used to pump or drive liquid into a manifold. According to various embodiments, and as illustrated in FIG. 25, each different liquid supply conduit (2502, 2504, 2506, 2508) is in fluid communication with either a syringe pump (2510, 2512, 2514, 2516) or a liquid supply vessel (2518, 2520, 2522, 2524) via a flow path of a three-way valve (2526, 2528, 2530, 2532, and illustrated as a circle around the junction of the conduit from the liquid supply vessel and the conduit between the immiscible-fluid-discrete-volume supply conduit 2534 and the respective, dedicated syringe pump). In some embodiments, when liquid is to be added to immiscible-fluid-discrete-volume supply conduit, a valve 2526 connects supply vessel 2518 and syringe pump 2510, and syringe pump 2510 withdraws liquid into through valve 2526. Valve 2526 is then switched to allow flow from syringe pump 2510 to immiscible-fluid-discrete-volume supply conduit 2534 through liquid supply conduit 2502 and syringe pump 2510 advances the liquid to the junction at the desired flow rate to add a desired volume of liquid to either the spacing fluid or an immiscible-fluid-discrete-volume. Conduit 2502 between valve 2526 and immiscible-fluid-discrete-volume conduit 2534 may be primed with spacing fluid drawn from the immiscible-fluid-discrete-volume conduit 2534 by syringe pump 2510 before withdrawing the liquid to be added from supply vessel 2518. This volume of spacing fluid is then added back to immiscible-fluid-discrete-volume supply conduit 2534 before the desired liquid can enter immiscible-fluid-discrete-volume supply conduit 2534.

A consideration in this regard can be the number of liquids to be introduced, in the case when a large number of samples are to be introduced into a tube. According to various embodiments, and as illustrated in FIGS. 26 and 27, one pump, which communicates with one or more valves that regulate the opening, closing, and/or diversion of channels or liquids in channels, can deliver a different liquid through each liquid-addition conduit to which it is connected. FIG. 26 illustrates a system the same as that illustrated in FIG. 25, except that instead of a dedicated syringe pump displacing a liquid, one pressure pump 2610 can flow the liquid in each conduit to add that liquid to the immiscible-fluid-discrete-volume supply conduit. In FIG. 26, a single pump 2610 can negatively or positively pressurize the conduits to which it is connected. As illustrated, pump 2610 is connected, via conduits and a .. three-way valve, to a liquid supply vessel (2518, 2520, 2522, and 2524) and to an immiscible-fluid-discrete-volume supply conduit 2534. Between the three-way valve and the single pump, a conduit contains a structure past which the liquid does not flow (2602, 2604, 2606, and 2608). Liquid-stopping structures 2602, 2604, 2606, and 2608 can be a liquid-impermeable membrane (porous or gas permeable), a diaphragm, an orifice, or a set of orifices which are large enough for air to flow through, but too small for the aqueous slug to wet and flow through. In some embodiments, an orifice could be configured to also allow the passage of oil, to remove any priming oil. Liquid-stopping structures 2602, 2604, 2606, and 2608 permit different usage rates for the different fluids, with a common refill action, rather than requiring a fluid monitor for each line as would be required if the tubes were to be controlled individually.

One method of operating the system in FIG. 26 follows. With three-way valve 2526 connecting immiscible-fluid-discrete-volume supply conduit 2534 to pressure pump 2610, pressure pump 2610 pressurizes supply conduit 2502 to withdraw spacing fluid from immiscible-fluid-discrete-volume supply conduit 2534 up to a predetermined point as determined and controlled by, for example, a system as described in FIG. 21. A controller (not shown) switches three-way valve 2526 to connect pump 2610 and liquid supply vessel 2518. The controller causes pump 2610 to pressurize the conduits between it and supply vessel 2518 to flow liquid from supply vessel 2518 through the conduits toward pump 2610 up to liquid-stop structure 2602. The controller switches valve 2526 to connect immiscible-fluid-discrete-volume supply conduit 2534 and pump 2610 and causes pump 2610 to pressurize the conduits between it and immiscible-fluid-discrete-volume supply conduit 2534 to flow liquid from liquid-stop structure 2602 toward immiscible-fluid-discrete-volume supply conduit 2534. Three-way valve 2526 meters the amount of liquid added to immiscible-fluid-discrete-volume supply conduit 2534. In some embodiments, valve 2526 may have at least an additional position in which it can prevent fluid communication between all three tubes connected to it.

Another method of operating the system illustrated in FIG. 26 is to prime the line between the pump and the immiscible-fluid-discrete-volume supply conduit with the desired "add" liquid, prior to the flow of immiscible-fluid-discrete-volumes in the immiscible-fluid-discrete-volume supply conduit. In contrast to the previously described method, this method allows the immediate introduction of the "add" liquid to the immiscible-fluid-discrete-volume supply conduit, rather than having to first flow the spacing fluid in the "add" conduit to the immiscible-fluid-discrete-volume supply conduit.

According to various embodiments, and as illustrated in FIG. 27, single pressure pump 2610 described in conjunction with FIG. 26 can directly pressurize liquid supply vessels 2518, 2520, 2522, and 2524. Thus, valve 2702, which no longer needs to be at least a three way valve, meters the amount of liquid added to immiscible-fluid-discrete-volume supply conduit 2524.

Another method to pump or transport liquids through the manifold can involve displacement of liquids localized near the junction of the addition conduit and the immiscible-fluid-discrete-volume supply conduit. Compression of the tube with a solenoid, roller, or other mechanism, may more dispense a more consistent volume of addition liquid each time in comparison to metered flow of pressurized liquid. Displacing the liquid in the addition conduit by compressing or pinching the tubes with rollers to press the tubes down is one example. As a roller is moved along the tube the pinching action of the tube can be used to push liquid into or out of the manifold. In some embodiments, a roller can be positioned in a range from about 2 inches to 10 inches away from the junction. Another is actuating a solenoid against the tube. In some embodiments, a solenoid can be positioned in a range from about 1 inch to about 1 foot away from the junction. Positioning may depend on conduit thickness and the actuation force of a selected solenoid.

Pumping and routing techniques, according to various embodiments of the present teachings, can eliminate the need for re-dipping a tube tip into different supply or other wells. This in one regard can minimize contamination. In various embodiments, the disclosed techniques can be performed in a totally enclosed, vacuum-sealed or otherwise isolated system, such that the introduction of air bubbles can be avoided.

In FIG. 28, an apparatus for and method of joining two streams of immiscible-fluid-discrete-volumes are illustrated In some embodiments, additional spacing fluid can be added and slug spacing can be monitors to control alignment of a slug from each stream with a slug from the other stream. In some embodiments, the two slug streams can be a set of samples and a set of primers which are loaded from two XYZ mechanisms from trays, permitting complete flexibility in the sorting and matching of primers and samples.

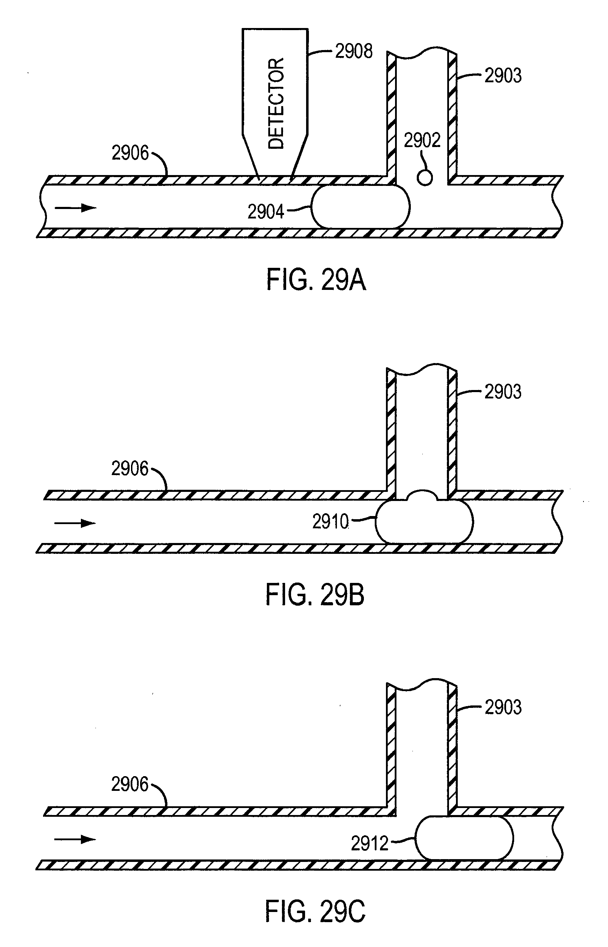

FIGS. 29A, B, and C illustrate a progression of directing a microdroplet 2902 of aqueous liquid into a slug 2904 flowing in a main conduit 2906, as controlled with feed back from a detector 2908 of the position of the slug 2904. Knowing the distance of detector 2908 from a junction of a conduit 2903 containing microdroplet 2902, and the speed of slug 2904, the movement of microdroplet 2902 toward slug 2904 can be timed such that while slug 2904 is in the junction of conduits 2903 and 2906, microdroplet 2902 will contact and coalesce forming a larger discrete volume 2910, which as it moves past the junction will form a supplemented slug 2910.

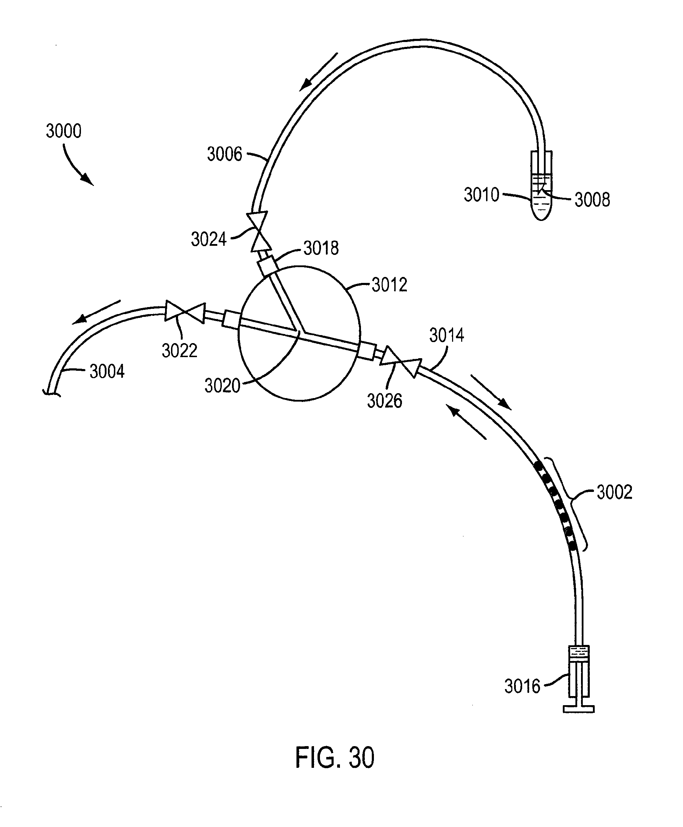

Fig. 30 depicts a system 3000 for generating a set of immiscible-fluid-discrete-volumes 3002 and subsequently pushing the set of immiscible-fluid-discrete-volumes 3002 into a downstream processing conduit 3004. System 3000 can be used to carry out a method wherein an immiscible-fluid-discrete-volume-forming conduit 3006, comprising an injection tip 3008, is manipulated in a two fluid-containing vessel 3010 to form spaced apart aqueous immiscible-fluid-discrete-volumes having spacing fluid disposed between adjacent immiscible-fluid-discrete-volumes in the set. A set of immiscible-fluid-discrete-volumes generated in immiscible-fluid-discrete-volume-forming conduit 3006 can be pulled through a Y-junction body 3012 and into a temporary holding conduit 3014 by negative pressure created in temporary holding conduit 3014 via a syringe pump 3016, although other suitable types of pumps can be used. By reversing the action of syringe pump 3016, a set of immiscible-fluid-discrete-volumes 3002 that have been pulled into temporary holding conduit 3014 can then be pushed through and out of the temporary holding conduit 3014, through Y-junction body 3012, and downstream into processing conduit 3004.

As shown in Fig. 30, each of conduits 3004, 3006, and 3014 can be connected to Y-junction body 3012 through a ferrule, such as ferrule 3018 as shown. In some embodiments, Y-junction body 3012 can comprise a valve-free junction 3020, as shown. In other embodiments, Y-junction body 3012 can be provided with a valve, for example, a multi-channel diverter valve such as valve 400 shown in FIGS. 4 and 5.

Using the system shown in Fig. 30, many different sets or batches of aqueous immiscible-fluid-discrete-volumes can be generated, temporarily held, and pushed into a downstream processing tube. For example, if the holding tube accommodates 100 aqueous immiscible-fluid-discrete-volumes spaced apart therein by spacing fluid, and 1000 immiscible-fluid-discrete-volumes are desired, 10 processes can be carried out wherein, for example, the temporary holding conduit 3014 is filled with a set of 100 immiscible-fluid-discrete-volumes, a diverter and/or valve in the Y-junction body 3012 is switched to cause a fluid communication with a downstream processing conduit, and each set of 100 immiscible-fluid-discrete-volumes are pushed through the Y-junction body 3012 into fluid processing conduit 3004, one set at a time. Automated control of valving, if provided, within the Y-junction body 3012 can facilitate the synchronization of valve actuation so that when syringe pump 3016 applies positive pressure to push fluid, the set of immiscible-fluid-discrete-volumes in temporary holding tube 3014 can only exit the Y-junction body 3012 to the downstream fluid processing conduit 3004.

In order to have great control over very small fluid volumes, a conventional syringe pump can be used as syringe pump 3016, and in some embodiments, gearing can be implemented to gear down the otherwise conventional syringe pump to accommodate small movements of finite volumes of fluid. In some embodiments, a reciprocating pump can be used with appropriate gearing to provide both negative pressure and positive pressure, alternately.

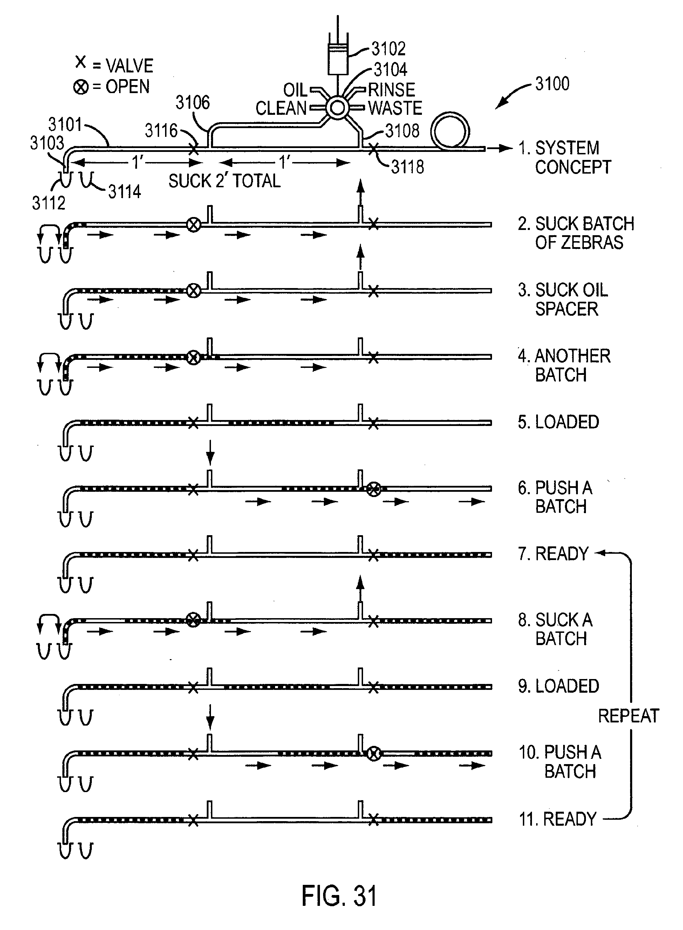

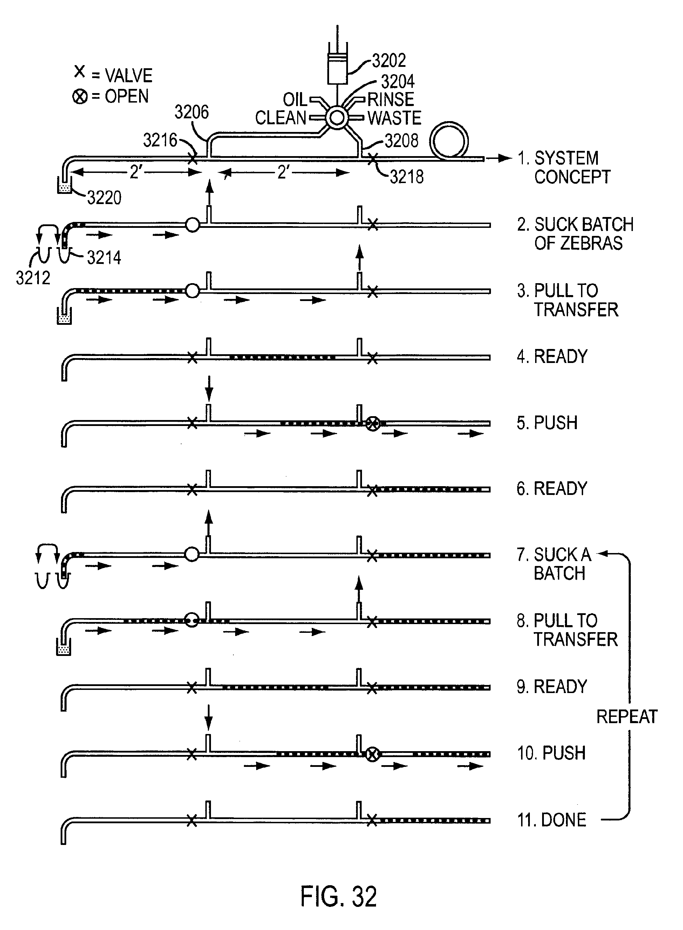

According to various embodiments, an immiscible-fluid-discrete-volume can be generated in an immiscible-fluid-discrete-volume-forming conduit, and spaced apart by spacing fluid, according to any of the various methods described herein. To minimize and/or eliminate the formation of air bubbles in an immiscible-fluid-discrete-volume-forming conduit, and to minimize or eliminate merging of adjacent spaced-apart immiscible-fluid-discrete-volumes, methods of pushing a pattern of immiscible-fluid-discrete-volumes and spacing fluid through a conduit can be used after the immiscible-fluid-discrete-volumes are generated. In so doing, a pattern of immiscible-fluid-discrete-volumes can be moved through a processing conduit without the use of negative pressure. An exemplary system for pushing a pattern of immiscible-fluid-discrete-volumes through a conduit, after the pattern is formed, is depicted in Figs. 31 and 32.

As shown in Fig. 31 a method is provided that can involve the generation of a relatively small number of immiscible-fluid-discrete-volumes in a pattern, spaced apart from one another by an average distance by a spacing fluid, which set of immiscible-fluid-discrete-volumes can then be separated from a subsequent set of immiscible-fluid-discrete-volumes to achieve a separation distance between sets that is greater than the average distance between immiscible-fluid-discrete-volumes in a single set. Once each set of immiscible-fluid-discrete-volumes is generated, the set can be pushed, rather than pulled, into a main flow path or main processing conduit, using positive pressure, thereby reducing and/or eliminating the creation of air bubbles or merging of adjacent immiscible-fluid-discrete-volumes in the processing conduit. A system 3100, as depicted in Fig. 31, can be used to carry out such a method.

As shown in Fig. 31, system 3100 can include a pump 3102 operatively connected to a selector valve 3104 that can be manipulated to perform a number of actions in an immiscible-fluid-discrete-volume-forming conduit 3101. As shown in Fig. 31, selector valve 3104 is operatively connected to ports 3106 and 3108 which can each be used as an inlet port and an outlet port, depending upon a desired action selected. Immiscible-fluid-discrete-volume-forming conduit 3101 is provided with valves 3116 and 3118 adjacent ports 3106 and 3108, respectively.

Fig. 31 shows a total of 11 method steps that can be used to carry out an immiscible-fluid-discrete-volume-forming operation as described above, and depicts the various states of valves 3116 and 3118, and the direction of flow through ports 3106 and 3108, in each step. In the first step shown at the top of Fig. 31, both valves 3116 and 3118 are closed and an intake tip 3103 of immiscible-fluid-discrete-volume-forming conduit 3101 is positioned within a spacing fluid vessel 3112. Next, valve 3116 is opened while valve 3118 remains closed, and pump 3102 is actuated to draw fluid into port 3108. The drawing action is timed with an alternating disposition of intake tip 3103 back-and-forth between spacing fluid vessel 3112 and an aqueous immiscible-fluid-discrete-volune fluid vessel 3114. The alternating submersion of intake tip 3103 into the spacing fluid in vessel 3112 and the aqueous immiscible-fluid-discrete-volume fluid in vessel 3114, as described elsewhere herein, generates a pattern of aqueous immiscible-fluid-discrete-volumes in the immiscible-fluid-discrete-volume-forming conduit 3101, separated from one another by spacing fluid. The pattern of spaced aqueous immiscible-fluid-discrete-volumes in immiscible-fluid-discrete-volume-forming conduit 3101 is referred to herein as a zebra pattern.

After a first set of aqueous immiscible-fluid-discrete-volumes is formed in conduit 3101, for example as illustrated, 15 spaced-apart aqueous immiscible-fluid-discrete-volumes, tip 3103 is then held in spacing fluid vessel 3112 for a period of time sufficient to enable the uptake of a large spacing fluid spacer following the first set of 15 aqueous immiscible-fluid-discrete-volumes. The large spacer can be used to separate the first set of aqueous immiscible-fluid-discrete-volumes from a subsequent set of aqueous immiscible-fluid-discrete-volumes, as shown in the third and fourth steps depicted in Fig. 31.

After two complete sets of aqueous immiscible-fluid-discrete-volumes are generated in immiscible-fluid-discrete-volume-forming conduit 3101, intake tip 3103 is held in spacing fluid 3112 and valve 3116 is closed such that the first set of aqueous immiscible-fluid-discrete-volumes, but not the second set of aqueous immiscible-fluid-discrete-volumes, is located along conduit 3101 between port 3106 and 3108, as shown in the fifth step of the process identified in Fig. 31 as the first "Loaded" step. Once the first set of aqueous immiscible-fluid-discrete-volumes is loaded as shown in the fifth step, valve 3118 is opened and pump 3102 is configured along with selector valve 3104 to push spacing fluid through port 3106 into conduit 3101, and through and past valve 3118, as shown in the sixth step identified as the "Push a Batch" step. Once the first set of immiscible-fluid-discrete-volumes passes valve 3118, valve 3118 is closed and the first set of immiscible-fluid-discrete-volumes is ready for down-stream processing as shown in the seventh step identified as the "ready" step. Next, as depicted in steps 8-11, the second set of immiscible-fluid-discrete-volumes is pulled through open valve 3116 until it is positioned between ports 3106 and 3108, while at the same time a third set of immiscible-fluid-discrete-volumes is generated by the alternating disposition of intake tip 3103 in vessels 3112 and 3114. As shown in the ninth step identified as the "Loaded" step, once the second set of immiscible-fluid-discrete-volumes is positioned between ports 3106 and 3108, valve 3116 is closed and the second set of immiscible-fluid-discrete-volumes is pushed through valve 3118 (step 10 "Push a Batch") in the same manner that the first set of immiscible-fluid-discrete-volumes was pushed in the sixth step ("Push a Batch") described above. The method described in connection with Fig. 31 can be repeated so that multiple sets of immiscible-fluid-discrete-volumes can be pushed into a down-stream processing conduit under positive pressure, with each set being spaced apart from a subsequent set by a relatively large spacing fluid spacer.

An alternative method to that shown in Fig. 31 is depicted in Fig. 32 wherein a pump 3202, selector valve 3204, ports 3206 and 3208, and valves 3216 and 3218, are shown. In the method shown in connection with Fig. 32, the intake tip of the immiscible-fluid-discrete-volume-forming conduit can be disposed initially in a rinse fluid retained in vessel 3220 prior to being disposed alternately in a spacing fluid vessel 3212 and an aqueous immiscible-fluid-discrete-volume fluid vessel 3214. Such a rinse vessel can be used similarly in Fig 31. As shown in Fig. 32, the negative pressure used to initially uptake the spacing fluid and aqueous immiscible-fluid-discrete-volumes is drawn through upstream port 3206 as opposed to being drawn through downstream port 3208. Another difference between the system and method shown in Fig. 32 relative to the system and method shown in Fig. 31 is that a first set of aqueous immiscible-fluid-discrete-volumes is moved all the way to a ready position, identified as the sixth step shown ("Ready"), before a second set of immiscible-fluid-discrete-volumes is generated as shown in the seventh step identified as ("Suck a Batch").

As can be understood with reference to Figs. 30, 31, and 32, the present teachings provide, in some embodiments, a method comprising: applying a negative pressure to a conduit system comprising an intake tip; contacting the intake tip with a first fluid and a second fluid that is immiscible with the first fluid, while applying the negative pressure, to draw the first fluid and the second fluid into the conduit system and form a set of discrete volumes of the first fluid spaced apart from one another by the second fluid, the set moving in a first direction in the conduit system; and thereafter, applying a positive pressure to the conduit system to push the set of discrete volumes in the conduit system. In some embodiments, the method can comprise applying a positive pressure that causes the set to move in the first direction. In some embodiments, the method can comprise applying a positive pressure that causes the set to move in the conduit system in a second direction that is opposite the first direction. In some embodiments the method can comprise applying the negative pressure to the conduit system until the set moves past a first diverter and the method can further comprise then changing a position of the diverter before applying the positive pressure, for example, to change the pathway of the set. In some embodiments, the method can comprise applying the negative pressure to the conduit system until the set moves past a valve and a port, and the method can further comprise then closing the valve, and furthermore, the applying of positive pressure can comprise applying a positive pressure through the port. In some embodiments, the method can comprise applying the negative pressure with a reversible pump, and reversing the action of the reversible pump to apply the positive pressure. In some embodiments, the contacting further comprises applying an electrowetting force to move at least one of the first fluid and the second fluid to a location adjacent the intake tip.

According to various embodiments of the present teachings, a method is provided that comprises: alternately introducing a first fluid and a second fluid, that is immiscible with the first fluid, into a conduit, to form a set of immiscible discrete volumes of the second fluid, each immiscible discrete volume of the set being separated from one or more other immiscible discrete volumes of the set by the first fluid, the set comprising a first end and a second end; moving the set of immiscible discrete volumes in a first direction by withdrawing from the conduit, some of the first fluid from the first end of the set; and moving the set in the first direction by adding to the conduit, more first fluid at the second end of the set. In some embodiments, the method can involve processing a first fluid that comprises an oil and a second fluid that comprises an aqueous liquid, for example, an aqueous sample that is immiscible in the oil. In some embodiments, the method can further comprise moving the set past a valve in the conduit and closing the valve before moving the set in the first direction by adding to the conduit more first fluid at the second end of the set. In some embodiments, closing a valve can comprise rotating a rotary valve as described herein, for example, in connection with Figs. 3-5.

## Claims

1. A method of splitting a discrete volume of a liquid contained in a conduit into two or more smaller discrete volumes of the liquid, the method comprising:
connecting to a first displacement pump (1518) having a first cross-sectional area a first conduit for receiving a first, smaller discrete volume of a first liquid;
connecting to a second displacement pump (1530) having a second cross-sectional area a second conduit for receiving a second, smaller discrete volume of the first liquid;
supplying a discrete volume of a first liquid in contact with a second liquid with which the first liquid is immiscible through a third conduit to a junction (1502) of at least the first and second conduits; and
moving a first displacement mechanism (1519) of the first displacement pump (1518) and a second displacement mechanism (1531) of the second displacement pump (1530) in a same first direction at the same time and at the same speed, thereby splitting the discrete volume of the first liquid into at least the first and second smaller discrete volumes of the first liquid;
wherein the ratio of the volume of the first smaller discrete volume of the first liquid to the volume of the second smaller discrete volume of the first liquid is based on the ratio of the cross-sectional area of the first displacement pump (1518) to the cross-sectional area of the second displacement pump (1530).

2. The method of claim 1, wherein moving the first and second displacement mechanisms (1519, 1531) in a first direction at the same time comprises moving a linkage (1532) attached to at least the first and second displacement pumps (1518, 1530).

3. The method of claim 2, further comprising:
connecting the common port of a first three-way valve (1510) to the first displacement pump (1518);
connecting the first of two input/output ports of the first three-way valve (1510) to the first conduit;
connecting the second of two input/output ports of the first three-way valve (1510) to a third conduit;
connecting the common port of a second three-way valve (1522) to the output of the second displacement pump (1530);
connecting the first of two input/output ports of the second three-way valve (1522) to the second conduit;
connecting the second of two input/output ports of the second three-way valve (1522) to a fourth conduit;
after moving the linkage in a first direction, moving the linkage (1532) in a second direction opposite the first direction, thereby displacing a first and second volume of the second liquid into the third and fourth conduits, respectively.

4. The method of claim 2, wherein the supplying to a junction (1502) comprises:
detecting the speed of the discrete volume of the first fluid, which is flowing in a third conduit connected to the junction of at least the first and second conduits, at a known distance from the junction (1502); and
stopping the flow of the discrete volume of the first fluid at the junction (1502).

5. An apparatus comprising:
a first displacement pump (1518) having a first cross-sectional area and a first displacement mechanism (1519);
a first conduit for receiving a first, smaller discrete volume of a first liquid connected to the first displacement pump (1518);
a second displacement pump (1530) having a second cross-sectional area and a second displacement mechanism (1531);
a second conduit for receiving a second, smaller discrete volume of the first liquid connected to the second displacement pump (1530);
a third conduit for containing a discrete volume of a first liquid in a second liquid with which the first liquid is immiscible supplying to a junction (1502) of at least the first and second conduits; and
wherein when said first displacement mechanism (1519) of the first displacement pump (1518) and said second displacement mechanism (1531) of the second displacement pump (1530) are moved in a same first direction at the same time and at the same speed, the discrete volume of the first liquid splits into at least the first and second smaller discrete volumes of the first liquid, and the ratio of the volume of the first smaller discrete volume of the first liquid to the volume of the second smaller discrete volume of the first liquid is based on the ratio of the cross-sectional area of the first displacement pump (1518) to the cross-sectional area of the second displacement pump (1530).

6. The apparatus of claim 5, further comprising a linkage (1532) attached to at least the first and second displacement pumps (1518, 1530), wherein the first and second displacement mechanisms (1519, 1531) are moved in the same first direction when the linkage (1532) is moved in the same first direction.

7. The apparatus of claim 6, further comprising:
a first three-way valve (1510) having a common port, and at least two input/output ports, wherein the common port of the first three-way valve (1510) is connected to the output of the first displacement pump (1518), the first of two input/output ports of the first three-way valve (1510) is connected to the first conduit, and the second of two input/output ports of the first three-way valve (1510) is connected to a fourth conduit;
a second three-way valve (1522) having a common port and at least two input/output ports, wherein the common port of a second three-way valve (1522) is connected to the output of the second displacement pump (1530), the first of two input/output ports of the second three-way valve (1522) is connected to the second conduit, and the second of two input/output ports of the second three-way valve (1522) is connected to a fifth conduit;
wherein when the linkage (1532) is moved in a second direction opposite the first direction, the first displacement pump (1518) displaces a first volume of the second liquid into the fourth conduit and the second displacement pump (1530) displaces a second volume of the second liquid into the fifth conduit.

8. The apparatus of claim 6, further comprising a detector adjacent the third conduit and operatively coupled to the motor.

9. The apparatus of claim 7, wherein at least one of the first and second three-way valves (1510, 1522) is a rotary three-way valve.

## Patentansprüche

1. Verfahren zur Spaltung eines in einer Leitung enthaltenen diskreten Flüssigkeitsvolumens in zwei oder mehr kleinere diskrete Volumina der Flüssigkeit, umfassend:
Anschließen einer ersten Leitung zur Aufnahme eines ersten, kleineren diskreten Volumens einer ersten Flüssigkeit an eine Verdrängerpumpe (1518), die eine erste Querschnittsfläche aufweist;
Anschließen einer zweiten Leitung zur Aufnahme eines zweiten, kleineren diskreten Volumens der ersten Flüssigkeit an eine zweite Verdrängerpumpe (1530), die eine zweite Querschnittsfläche aufweist;
Zuführen eines diskreten Volumens einer ersten Flüssigkeit in Kontakt mit einer zweiten Flüssigkeit, mit der die erste Flüssigkeit unvermischbar ist, über eine dritte Leitung zu einer Anschlussstelle (1502) mindestens der ersten und zweiten Leitung; und
gleichzeitiges Verschieben eines ersten Verdrängungsmechanismus (1519) der ersten Verdrängerpumpe (1518) und eines zweiten Verdrängungsmechanismus (1531) der zweiten Verdrängerpumpe (1530) in dieselbe erste Richtung und mit derselben Geschwindigkeit, wodurch das diskrete Volumen der ersten Flüssigkeit in mindestens das erste und zweite kleinere diskrete Volumen der ersten Flüssigkeit aufgespalten wird;
wobei das Volumenverhältnis des ersten kleineren diskreten Volumens der ersten Flüssigkeit zum Volumen des zweiten kleineren diskreten Volumens der ersten Flüssigkeit auf dem Verhältnis der Querschnittsfläche der ersten Verdrängerpumpe (1518) zur Querschnittsfläche der zweiten Verdrängerpumpe (1530) basiert.

2. Verfahren nach Anspruch 1, wobei das gleichzeitige Verschieben des ersten und zweiten Verdrängungsmechanismus (1519, 1531) in eine erste Richtung das Verschieben einer an mindestens die erste und zweite Verdrängerpumpe (1518, 1530) angeschlossenen Verbindung (1532) umfasst.

3. Verfahren nach Anspruch 2, ferner umfassend:
Anschließen der gemeinsamen Verbindung eines ersten Dreiwegeventils (1510) an die erste Verdrängerpumpe (1518);
Anschließen der ersten von zwei Input/Output-Verbindungen des ersten Dreiwegeventils (1510) an die erste Leitung;
Anschließen der zweiten von zwei Input/Output-Verbindungen des ersten Dreiwegeventils (1510) an eine dritte Schaltung;
Anschließen der gemeinsamen Verbindung eines zweiten Dreiwegeventils (1522) an den Ausgang der zweiten Verdrängerpumpe (1530);
Anschließen der ersten von zwei Input/Output-Verbindungen des zweiten Dreiwegeventils (1522) an die zweite Leitung;
Anschließen der zweiten von zwei Input/Output-Verbindungen des zweiten Dreiwegeventils (1522) an eine vierte Leitung;
nach dem Verschieben der Verbindung in eine erste Richtung, Verschieben der Verbindung (1532) in eine zweite, der ersten Richtung entgegengesetzte Richtung, wodurch ein erstes und ein zweites Volumen der zweiten Flüssigkeit jeweils in die dritte und vierte Leitung verdrängt wird.

4. Verfahren nach Anspruch 2, wobei das Zuführen zu einer Anschlussstelle (1502) umfasst:
Ermitteln der Geschwindigkeit des diskreten Volumens der ersten Flüssigkeit, die in einer an die Anschlussstelle mindestens der ersten und zweiten Leitung angeschlossenen dritten Leitung fließt, in einem bekannten Abstand zur Anschlussstelle (1502); und
Aufhalten des Flusses des diskreten Volumen der ersten Flüssigkeit an der Anschlussstelle (1502).

5. Vorrichtung, umfassend:
eine erste Verdrängerpumpe (1518), die eine erste Querschnittsfläche und einen ersten Verdrängungsmechanismus (1519) aufweist;
eine an die erste Verdrängerpumpe (1518) angeschlossene erste Leitung zur Aufnahme eines ersten, kleineren diskreten Volumens einer ersten Flüssigkeit;
eine zweite Verdrängerpumpe (1530), die eine zweite Querschnittsfläche und einen zweiten Verdrängungsmechanismus (1531) aufweist;
eine an die zweite Verdrängerpumpe (1530) angeschlossene zweite Leitung zur Aufnahme eines zweiten, kleineren diskreten Volumens einer ersten Flüssigkeit;
eine dritte Leitung zur Aufnahme eines eines diskreten Volumens einer ersten Flüssigkeit in einer zweiten Flüssigkeit, mit der die erste Flüssigkeit mischbar ist, zu einer Anschlussstelle (1502) mindestens der ersten und zweiten Leitung; und
wobei wenn der erste Verdrängungsmechanismus (1519) der ersten Verdrängerpumpe (1518) und der zweite Verdrängungsmechanismus (1531) der zweiten Verdrängerpumpe (1530) gleichzeitig in dieselbe erste Richtung und mit derselben Geschwindigkeit verschoben werden, das diskrete Volumen der ersten Flüssigkeit in mindestens das erste und zweite kleinere diskrete Volumen der ersten Flüssigkeit aufgespalten wird, und das Volumenverhältnis des ersten kleineren diskreten Volumens der ersten Flüssigkeit zum Volumen des zweiten kleineren diskreten Volumens der ersten Flüssigkeit auf dem Verhältnis der Querschnittsfläche der ersten Verdrängerpumpe (1518) zur Querschnittsfläche der zweiten Verdrängerpumpe (1530) basiert.

6. Vorrichtung nach Anspruch 5, ferner umfassend eine an mindestens die erste und zweite Verdrängerpumpe (1518, 1530) angeschlossene Verbindung (1532) angeschlossen ist, wobei der erste und zweite Verdrängungsmechanismus (1519, 1531) in dieselbe erste Richtung verschoben werden, wenn die Verbindung (1532) in dieselbe erste Richtung verschoben wird.

7. Vorrichtung nach Anspruch 6, ferner umfassend:
ein erstes Dreiwegeventil (1510), die eine gemeinsame Verbindung sowie mindestens zwei Intput/Output-Verbindungen aufweist, wobei die gemeinsame Verbindung des ersten Dreiwegeventils (1510) an den Ausgang der ersten Verdrängerpumpe (1518), die erste der beiden Input/Output-Verbindungen des ersten Dreiwegeventils (1510) an die erste Leitung, und die zweite der beiden Input/Output-Verbindungen des ersten Dreiwegeventils (1510) an eine vierte Leitung angeschlossen ist;
ein zweites Dreiwegeventil (1522), die eine gemeinsame Verbindung sowie mindestens zwei Intput/Output-Verbindungen aufweist, wobei die gemeinsame Verbindung des zweiten Dreiwegeventils (1522) an den Ausgang der zweiten Verdrängerpumpe (1530), die erste der beiden Input/Output-Verbindungen des zweiten Dreiwegeventils (1522) an die zweite Leitung, und die zweite der beiden Input/Output-Verbindungen des zweiten Dreiwegeventils (1522) an eine fünfte Leitung angeschlossen ist;
wobei wenn die Verbindung (1532) in eine zweite, der ersten Richtung entgegengesetzte Richtung verschoben wird, die erste Verdrängerpumpe (1518) ein erstes Volumen der zweiten Flüssigkeit in die vierte Leitung, und die zweite Verdrängerpumpe (1530) ein zweites Volumen der zweiten Flüssigkeit in die fünfte Leitung verdrängt.

8. Vorrichtung nach Anspruch 6, ferner umassend einen neben der dritten Leitung und operativ an den Motor gekoppelten Detektor.

9. Vorrichtung nach Anspruch 7, wobei mindestens eines der ersten und zweiten Dreiwegeventile (1510, 1522) ein rotierendes Dreiwegeventil ist.

## Revendications

1. Procédé de fractionnement d'un volume discret d'un liquide contenu dans un conduit en au moins deux volumes discrets plus petits du liquide, le procédé comprenant les opérations consistant à :
relier à une première pompe à déplacement (1518) ayant une première surface en coupe transversale un premier conduit pour recevoir un premier volume discret, plus petit, d'un premier liquide ;
relier à une seconde pompe à déplacement (1530) ayant une seconde surface en coupe transversale un deuxième conduit pour recevoir un second volume discret, plus petit, du premier liquide ;
fournir un volume discret d'un premier liquide en contact avec un second liquide avec lequel le premier liquide est immiscible à travers un troisième conduit à une jonction (1502) d'au moins les premier et deuxième conduits ; et
mettre en mouvement un premier mécanisme de déplacement (1519) de la première pompe à déplacement (1518) et un second mécanisme de déplacement (1531) de la seconde pompe à déplacement (1530) dans une même première direction au même moment et à la même vitesse, fractionnant par là le volume discret du premier liquide en au moins les premier et second volumes discrets plus petits du premier liquide ;
le rapport du volume du premier volume discret plus petit du premier liquide au volume du second volume discret plus petit du premier liquide étant basé sur le rapport de la surface en coupe transversale de la première pompe à déplacement (1518) à la surface en coupe transversale de la seconde pompe à déplacement (1530).

2. Procédé selon la revendication 1, dans lequel le déplacement des premier et second mécanismes de déplacement (1519, 1531) dans une première direction au même moment comprend le déplacement d'une liaison (1532) fixée à au moins les première et seconde pompes à déplacement (1518, 1530).

3. Procédé selon la revendication 2, comprenant en outre les opérations consistant à :
relier l'orifice commun d'une première soupape à trois voies (1510) à la première pompe à déplacement (1518) ;
relier le premier de deux orifices d'entrée/sortie de la première soupape à trois voies (1510) au premier conduit ;
relier le second de deux orifices d'entrée/sortie de la première soupape à trois voies (1510) à un troisième conduit ;
relier l'orifice commun d'une seconde soupape à trois voies (1522) à la sortie de la seconde pompe à déplacement (1530) ;
relier le premier de deux orifices d'entrée/sortie de la seconde soupape à trois voies (1522) au deuxième conduit ;
relier le second de deux orifices d'entrée/sortie à la seconde soupape à trois voies (1522) à un quatrième conduit ;
après déplacement de la liaison dans une première direction, déplacer la liaison (1532) dans une seconde direction opposée à la première direction, déplaçant par là un premier et un second volume du second liquide dans les troisième et quatrième conduits respectivement.

4. Procédé selon la revendication 2, dans lequel la fourniture à une jonction (1502) comprend les opérations consistant à :
détecter la vitesse du volume discret du premier fluide, qui circule dans un troisième conduit relié à la jonction d'au moins les premier et deuxième conduits, à une distance connue de la jonction (1502) ; et
arrêter l'écoulement du volume discret du premier fluide à la jonction (1502).

5. Appareil comprenant
une première pompe à déplacement (1518) ayant une première surface en coupe transversale et un premier mécanisme de déplacement (1519) ;
un premier conduit pour recevoir un premier volume discret, plus petit, d'un premier liquide, relié à la première pompe à déplacement (1518) ;
une seconde pompe à déplacement (1530) ayant une seconde surface en coupe transversale et un second mécanisme de déplacement (1531) ;
un deuxième conduit pour recevoir un second volume discret, plus petit, du premier liquide, relié à la seconde pompe à déplacement (1530) ;
un troisième conduit pour contenir un volume discret d'un premier liquide dans un second liquide avec lequel le premier liquide est immiscible de fourniture à une jonction (1502) d'au moins les premier et deuxième conduits ; et
lorsque ledit premier mécanisme de déplacement (1519) de la première pompe à déplacement (1518) et ledit second mécanisme de déplacement (1531) de la seconde pompe à déplacement (1530) sont déplacés dans la même direction au même moment et à la même vitesse, le volume discret du premier liquide est fractionné en au moins les premier et second volumes discrets, plus petits, du premier liquide, et le rapport du volume du premier volume discret, plus petit, du premier liquide, au volume du second volume discret, plus petit, du premier liquide est basé sur le rapport de la surface en coupe transversale de la première pompe à déplacement (1518) à la surface en coupe transversale de la seconde pompe à déplacement (1530).

6. Appareil selon la revendication 5, comprenant en outre une liaison (1532) fixée à au moins les première et seconde pompes à déplacement (1518, 1530), les premier et second mécanismes de déplacement (1519, 1931) étant déplacés dans la même direction lorsque la liaison (1532) est déplacée dans la même première direction.

7. Appareil selon la revendication 6, comprenant en outre :
une première soupape à trois voies (1510) ayant un orifice commun et au moins deux orifices d'entrée/sortie, l'orifice commun de la première soupape à trois voies (1510) étant relié à la sortie de la première pompe à déplacement (1518), le premier de deux orifices d'entrée/sortie de la première soupape à trois voies (1510) étant relié au premier conduit et le second de deux orifices d'entrée/sortie de la première soupape à trois voies (1510) étant relié à un quatrième conduit ;
une seconde soupape à trois voies (1522) ayant un orifice commun et au moins deux orifices d'entrée/sortie, l'orifice commun d'une seconde soupape à trois voies (1522) étant relié à la sortie de la seconde pompe à déplacement (1530), le premier de deux orifices d'entrée/sortie de la seconde soupape à trois voies (1522) étant relié au deuxième conduit et le second de deux orifices d'entrée/sortie de la seconde soupape à trois voies (1522) étant relié au cinquième conduit ;
lorsque la liaison (1532) est déplacée dans une seconde direction opposée à la première direction, la première pompe à déplacement (1518) déplace un premier volume du second liquide dans le quatrième conduit et la seconde pompe à déplacement (1530) déplace un second volume du second liquide dans le cinquième conduit.

8. Appareil selon la revendication 6, comprenant en outre un détecteur adjacent au troisième conduit et couplé de manière fonctionnelle au moteur.

9. Appareil selon la revendication 7, dans lequel au moins l'une des première et seconde soupapes à trois voies (1510, 1522) est une soupape à trois voies tournantes.
